# EUROPEAN PATENT SPECIFICATION

(11) **EP 1 443 970 B1**
(45) Date of publication and mention of the grant of the patent: **20.01.2010**
(21) Application number: 01996590.4
(22) Date of filing: 15.11.2001
(51) Int. Cl.: A61K 48/00, C12N 15/85

(54) **PROMOTERS EXHIBITING ENDOTHELIAL CELL SPECIFICITY AND METHODS OF USING SAME**
ENDOTHELZELLSPEZIFITÄT ZEIGENDE PROMOTOREN UND VERFAHREN ZU DEREN VERWENDUNG
PROMOTEURS PRESENTANT UNE SPECIFICITE POUR DES CELLULES ENDOTHELIALES ET LEURS PROCEDES D'UTILISATION

(30) Priority: 17.11.2000 US 248582 P
(43) Date of publication of application: 11.08.2004
(60) Divisional application: 09174998.6
(73) Proprietor: Vascular Biogenics Ltd., 60376 Or Yehuda (IL)
(72) Inventor: HARATS, Dror, 53 765 Ramat Gan (IL); BLOOM, Nira, 45309 Hod Hasharon (IL)
(74) Representative: Machtalère, Georges
(86) International application number: PCT/IL2001/001059
(87) International publication number: WO 2002/040629

(56) References cited:
- US-A- 5 747 340
- US-A- 5 792 453
- MINCHENKO ALEXANDER ET AL: "Regulation of endothelin-1 gene expression in human microvascular endothelial cells by hypoxia and cobalt: Role of hypoxia responsive element" MOLECULAR AND CELLULAR BIOCHEMISTRY, vol. 208, no. 1-2, May 2000 (2000-05), pages 53-62, XP002305520 ISSN: 0300-8177
- SUN GUO ET AL: "Functional analysis of the preproendothelin-1 gene promoter in pulmonary epithelial cells and monocytes" BIOCHEMICAL AND BIOPHYSICAL RESEARCH COMMUNICATIONS, vol. 221, no. 3, 1996, pages 647-652, XP002305521 ISSN: 0006-291X
- LEE ET AL: 'Functional analysis of the endothelin-1 gene promoter' JOURNAL OF BIOLOGICAL CHEMISTRY vol. 265, no. 18, 25 June 1990, pages 10446 - 10450, XP002980772
- BU ET AL: 'Identification of an endothelial cell-specific regulatory region in the murine endothelin-1 gene' JOURNAL OF BIOLOGICAL CHEMISTRY vol. 272, no. 51, 19 December 1997, pages 32613 - 32622, XP002980773
- VARDA-BLOOM ET AL: 'Tissue-specific gene therapy directed to tumor angiogenesis' GENE THERAPY vol. 8, no. 11, June 2001, pages 819 - 827, XP002980774

### SEQUENCE LISTING

<110> Harats, Dror
<120> PROMOTERS EXHIBITING ENDOTHELIAL CELL SPECIFICITY, AND METHODS OF USING SAME
<130> 01/22752
<150> US 60/248,582
   <151> 2000-11-17
<160> 8
<170> PatentIn version 3.1
<210> 1
   <211> 1334
   <212> DNA
   <213> Mus musculus
<400> 1
   gtgtgtgtgt gtgtgtgtgt gtgtgtgtgt gtgtgtgtgt gtgtagtgta cttctgatcg      60
   gcgatactag ggagataagg atgtacctga caaaaccaca ttgttgttgt tatcattatt     120
   atttagtttt ccttccttgc taactcctga cggaatcttt ctcacctcaa atgcgaagta     180
   ctttagttta gaaaagactt ggtggaaggg gtggtggtgg aaaagtaggg tgatcttcca     240
   aactaatctg gttccccgcc cgccccagta gctgggattc aagagcgaag agtggggatc     300
   gtccccttgt ttgatcagaa agacataaaa ggaaaatcaa gtgaacaatg atcagcccca     360
   cctccacccc acccccctgc gcgcgcacaa tacaatctat ttaattgtac ttcatacttt    420
   tcattccaat ggggtgactt tgcttctgga gaaactcttg attcttgaac tctggggctg     480
   gcagctagca aaaggggaag cgggctgctg ctctctgcag gttctgcagc ggtctctgtc     540
   tagtgggtgt tttctttttc ttagccctgc ccctggattg tcagacggcg ggcgtctgcc     600
   tctgaagtta gccgtgattt cctctagagc cgggtcttat ctctggctgc acgttgcctg     660
   tgggtgacta atcacacaat aacattgttt agggctggaa taaagtcaga gctgtttacc     720
   cccactctat aggggttcaa tataaaaagg cggcggagaa ctgtccgagt cagacgcgtt     780
   cctgcaccgg cgctgagagc ctgacccggt ctgctccgct gtccttgcgc gctgcctccc     840
   ggctgcccgc gacgctttcg ccccagtgga agggccactt gctgaggacc gcgctgagat     900
   ctaaaaaaaa aacaaaaaac aaaaaacaaa aaaacccaga ggcgatcaga gcgaccagac     960
   accgtcctct tcgttttgca ttgagttcca tttgcaaccg agttttcttt ttttcctttt    1020
   tccccactct tctgacccct ttgcagaatg gattattttc ccgtgatctt ctctctgctg    1080
   ttcgtgactt tccaaggagc tccagaaaca ggtaggcgcc acttgcgaat ctttctactt    1140
   cagcgcagca gttatcgctt ctgttttcca cttttctttc tttcttttct ttcattcttt    1200
   cctttttatt tattttttta attactgaag ctccagcagc aagtgcctta caattaatta   1260
   acttctgtgt gaagcgaaag aaataaaacc cctgtttgaa tacagctgac tacaaccgag    1320
   tatcgcatag cttc                                                      1334
<210> 2
   <211> 96
   <212> DNA
   <213> Artificial sequence
<220>
   <223> Synthetic oligonucleotide
<400> 2
   gctagcgtac ttcatacttt tcattccaat ggggtgactt tgcttctgga gggtgacttt 60
   gcttctggag ccaatgggta cttcatactt ttcatt 96
<210> 3
   <211> 96
   <212> DNA
   <213> Artificial sequence
<220>
   <223> Synthetic oligonucleotide
<400> 3
   gctagcctcc agaagcaaag tcaccccatt ggaatgaaaa gtatgaagta caatgaaaag 60
   tatgaagtac ccattggctc cagaagcaaa gtcacc 96
<210> 4
   <211> 6
   <212> DNA
   <213> Artificial sequence
<220>
   <223> Nhe-1 restriction site
<400> 4
   gctagc 6
<210> 5
   <211> 6
   <212> DNA
   <213> Mus musculus
<220>
   <221> misc_feature
   <223> Hypoxia responsive element - E-box
<400> 5
   gcacgt 6
<210> 6
   <211> 44
   <212> DNA
   <213> Mus musculus
<220>
   <221> misc_feature
   <223> Murine endothelial specific enhancer elemet
<400> 6
   gtacttcata cttttcattc caatggggtg actttgcttc tgga 44
<210> 7
   <211> 143
   <212> DNA
   <213> Artificial sequence
<220>
   <223> A triplicate copy of a murine enhancer sequence originated from t he PPE-1 promoter
<400> 7
   gtacttcata cttttcattc caatggggtg actttgcttc tggagggtga ctttgcttct     60
   ggagccagta cttcatactt ttcattgtac ttcatacttt tcattccaat ggggtgactt    120
   tgcttctgga ggctagctgc cag                                            143
<210> 8
   <211> 47
   <212> DNA
   <213> Artificial sequence
<220>
   <223> EDC fragment
<400> 8
   ctggagggtg actttgcttc tggagccagt acttcatact tttcatt 47

## Description

The present invention relates to an isolated polynucleotide according to claim1, a nucleic acid construct according to claims 8, 31, a mammalian cell according to claims 10, 33, the use of a promoter functional in endothelial cells according to claim 11, the use of a nucleic acid construct according to claims 20, 34, 38, an isolated polynucleotide according to claim 26.

The present invention relates to isolated polynucleotide sequences exhibiting endothelial cell specific promoter activity, and methods of use thereof and, more particularly, to a modified-preproendothelin-1 (PPE-1) promoter which exhibits increased activity and specificity in endothelial cells. The invention further relates to modifications of the PPE promoter which enhance its expression in response to physiological conditions including hypoxia and angiogenesis.

Gene therapy is an emerging modality for treating inherited and acquired human diseases. Great efforts are directed towards developing methods for gene therapy of cancer, cardiovascular and peripheral vascular diseases, but there is still a major obstacle in effective and specific gene delivery. In general, the main limiting factor of gene therapy with a gene of interest using a recombinant viral vector as a shuttle is the ability to specifically direct the gene of interest to the target tissue. Adenoviruses that are used for this purpose are capable of infecting a large variety of cells with different affinities. Indeed, using a non-tissue specific promoter induces up to 95% of the expression of the gene of interest in the liver; hence regulation of expression is highly required. Further, it is currently infeasible to differentiate between normal vascular endothelia and developing vascular endothelia in a growing tumor when targeting a gene.

In both cancer development and vascular diseases, angiogenesis, the creation of new vessels, plays a central role. Hence, regulation of this process by gene therapy to the vascular endothelium can be tremendously important in inducing targeted therapy for these diseases.

High efficiency of the human preproendothelin-1 (PPE-1), delivered by retroviral vector, was obtained in endothelial cells (EC) in-vitro, and in transgenic animal models. However, the prior art does not teach use of this promoter for in-vivo gene therapy. The human PPE-1 lacks regulatory elements found in PPE-1 genes of other animals, most notably the mouse.

United States patent 5,747,340 teaches use of the murine PPE-1 promoter and portions thereof. However, this patent contains no hint or suggestion that an endothelial-specific enhancer can be employed to increase the level of expression achieved with the PPE promoter while preserving endothelial specificity. Further, this patent does not teach that the PPE-1 promoter is induced to higher levels of transcription under hypoxic conditions.

Sun Guo et al discloses a functional analysis of the preproendothelin-1 gene promoter in pulmonary epithelial cells and monocytes, Biochemical and Biophysical Research Communications, vol. 221, no. 3, 1996, pages 647-652.

Minchenko Alexander et al relates to regulation of endothelin1- gene expression in human microvascular endothelial cells by hypoxia and cobalt: Role of hypoxia responsive element, Molecular and Cellular Biochemistry, vol. 208, no. 1-2, May 2000 (2000-05), pages 53-62.

Bu et al concerns the identification of an endothelial cell-specific regulatory region in the murine endotheline-1 gene, Journal of Biological Chemistry, 19.12.1987, vol. 51, no. 51, pages 32613-32622.

Jäger et al discloses endothelial cell-specific transcriptional targeting from a hybrid long terminal repeat retrovirus vector containing human prepro-endothelin-1 promoter sequences, Journal of Virology, December 1999, vol. 73, no. 12, pages 9702-9709.

There is thus a widely recognized need for, and it would be highly advantageous to have, an improved endothelial cell specific promoter, methods of use thereof and cells transformed therewith devoid of the above limitations. The disclosed invention is expected to demonstrate added utility in treatment of cardiovascular disease, cancer and wound healing relative to previously know configurations.

The isolated polynucleotide is defined in claim 1, the nucleic acid construct is defined in claims 8 and 31, a mammalian cell is defined in claims 10 and 33, the use of a promoter functional in endothelial cells is defined in claim 11, the use of a nucleic acid construct is defined in claims 20, 34 and 38 and the isolated polynucleotide is defined in claim 26.

According to one aspect of the present invention there is provided an isolated polynucleotide functional as a promoter in eukaryotic cells. The isolated polynucleotide includes an enhancer element including at least two copies of the sequence set forth in SEQ ID NO:6.

According to another aspect of the present invention there is provided a method of expressing a nucleic acid sequence of interest, encoding an active RNA molecule or a protein such as an enzyme, reporter molecule and the like in endothelial cells. The method includes administering to a subject a construct which includes the nucleic acid sequence of interest positioned under the regulatory control of a promoter functional in eukaryotic cells. The construct further includes an enhancer element including at least one copy of the sequence set forth in SEQ ID NO:6.

According to yet another aspect of the present invention there is provided a method of regulating angiogenesis in a tissue. The method includes administering a nucleic acid construct including: (a) an endothelial cell specific promoter; (b) at least one copy of a hypoxia response element set forth in SEQ ID NO:5; and (c) a nucleic acid sequence encoding an angiogenesis regulator, the nucleic acid sequence being under regulatory control of the promoter and the hypoxia response element.

According to still another aspect of the present invention there is provided an isolated polynucleotide functional as a promoter in eukaryotic cells. The isolated polynucleotide includes an enhancer element including the sequence set forth in SEQ ID NO: 7.

According to an additional aspect of the present invention there is provided a method of regulating angiogenesis in a tissue. The method includes administering a nucleic acid construct including: (a) an endothelial cell specific promoter; (b) an enhancer element including the sequence set forth in SEQ ID NO: 7; (c) at least one copy of a hypoxia response element set forth in SEQ ID NO:5; and (d) a nucleic acid sequence encoding an angiogenesis regulator, the nucleic acid sequence being under regulatory control of the promoter, the enhancer element and the hypoxia response element.

According to still a further aspect of the present invention there is provided isolated polynucleotide functional as a promoter in eukaryotic cells, the isolated polynucleotide includes an enhancer element including at least one copy of the sequence set forth in SEQ ID NO:8.

According to still a further aspect of the present invention there is provided a method of expressing a nucleic acid sequence of interest in endothelial cells, the method includes administering to a subject a construct, the construct includes the nucleic acid sequence of interest positioned under the regulatory control of a promoter functional in eukaryotic cells, and an enhancer element including at least one copy of the sequence set forth in SEQ ID NO:8.

According to still another further aspect of the present invention there is provided isolated polynucleotide functional as a promoter in eukaryotic cells, the isolated polynucleotide includes an enhancer element including the sequence set forth in SEQ ID NO: 8.

According to further features in preferred embodiments of the invention described below, the enhancer element includes three copies of the sequence set forth in SEQ ID NO:6.

According to still further features in preferred embodiments of the invention the at least two copies of the sequence set forth in SEQ ID NO:6 are contiguous.

According to still further features in preferred embodiments of the invention the isolated polynucleotide further includes an endothelial specific promoter element.

According to still further features in preferred embodiments of the invention the endothelial specific promoter element includes at least one copy of the PPE-1 promoter.

According to still further features in preferred embodiments of the invention the isolated polynucleotide further includes a hypoxia response element.

According to still further features in preferred embodiments of the invention the hypoxia response element includes at least one copy of the sequence set forth in SEQ ID NO: 5.

According to still further features in preferred embodiments of the invention the enhancer element is as set forth in SEQ ID NO: 7.

According to still further features in preferred embodiments of the invention there is provided a nucleic acid construct including a claimed isolated polynucleotide and a nucleic acid sequence of interest, the nucleic acid sequence of interest being under regulatory control of the isolated polynucleotide.

According to still further features in preferred embodiments of the invention the nucleic acid sequence of interest is selected from the group consisting of VEGF, p55 and PDGF-BB.

According to still further features in preferred embodiments of the invention there is provided a mammalian cell transformed with a claimed isolated polynucleotide.

According to still further features in preferred embodiments of the invention the promoter exhibits endothelial cell specificity.

According to still further features in preferred embodiments of the invention the promoter is the PPE-1 promoter as set forth in SEQ ID NO: 1.

According to still further features in preferred embodiments of the invention administering is effected by a method selected from the group consisting of: (i) systemic in-vivo administration; (ii) ex-vivo administration to cells removed from a body of a subject and subsequent reintroduction of the cells into the body of the subject; and (iii) local in-vivo administration.

According to still further features in preferred embodiments of the invention the nucleic acid construct further includes an enhancer element including at least two copies of the sequence set forth in SEQ ID NO:6.

According to still further features in preferred embodiments of the invention the endothelial cell specific promoter includes at least one copy of the PPE-1 promoter.

According to still further features in preferred embodiments of the invention there is provided a nucleic acid construct including a claimed isolated polynucleotide and a nucleic acid sequence of interest, the nucleic acid sequence of interest being under regulatory control of the isolated polynucleotide.

According to still further features in the described preferred embodiments the enhancer element further includes at least one copy of the sequence set forth in SEQ ID NO:6.

According to still further features in the described preferred embodiments the enhancer element includes one copy of the sequence set forth in SEQ ID NO:8 and at least two copies of the sequence set forth in SEQ ID NO:6.

According to still further features in the described preferred embodiments the enhancer element further includes at least one copy of the sequence set forth in SEQ ID NO:6.

According to still further features in the described preferred embodiments the at least one copy includes two copies.

According to still further features in the described preferred embodiments the nucleic acid construct further includes an enhancer element including at least one copy of the sequence set forth in SEQ ID NO:8.

According to yet another further aspect of the present invention there is provided method of regulating angiogenesis in a tissue, the method comprising administering a nucleic acid construct including: (a) an endothelial cell specific promoter; (b) an enhancer element including at least one copy of the sequence set forth in SEQ ID NO:8; and (c) a nucleic acid sequence encoding an angiogenesis regulator, the nucleic acid sequence being under regulatory control of the promoter and the enhancer element.

According to still further features in the described preferred embodiments the enhancer element further includes at least one copy of the sequence set forth in SEQ ID NO:6.

According to still further features in the described preferred embodiments the enhancer element includes one copy of the sequence set forth in SEQ ID NO:8 and at least two copies of the sequence set forth in SEQ ID NO:6.

The present invention successfully addresses the shortcomings of the presently known configurations by providing improved isolated polynucleotide sequences with endothelial cell specificity, and methods of use thereof. The improvements in the sequence make feasible methods of treating a variety of diseases, disorders and conditions which were previously considered infeasible. Specifically, the improvements relate to increased specificity to endothelial cells, increased levels of expression of a sequence of interest and enhanced induction by conditions including ischemia and angiogenesis.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 is a histogram illustrating the effect of the enhancer element of the present invention on Luciferase expression in both bovine and human endothelial cell lines using the B2B cell line as a non-endothelial control.
FIG. 2 is a histogram illustrating endothelial specificity of a promoter of the present invention in an adenoviral vector on Luciferase expression in various cell lines.
FIGs. 3A and 3B are photomicrographs illustrating GFP expression under the control of Ad5PPE-1-3X of the present invention and an Ad5CMV control construct in the BAEC cell line.
FIG. 4 is histogram of % apoptosis induced by pACPPE-1-3Xp55, pACPPE-1-3XLuciferase and pCCMVp55 in endothelial and non-endothelial cells.
FIG. 5 is a histogram illustrating the effect of introducing an enhancer element according to the present invention into a promoter construct on hypoxia response.
FIG. 6 is a histogram illustrating the effect of introducing an enhancer element according to the present invention into a promoter of an adenovector construct on hypoxia response.
FIG. 7 is a histogram illustrating the effect of introducing an enhancer element according to the present invention into a promoter on levels of expression in bovine and human endothelial cell lines.
FIG. 8 is a histogram illustrating levels of expression of a reporter gene observed in various organs after injection of an adenoviral construct containing either an endothelial promoter (PPE-1) or a control (CMV) promoter;
FIGs. 9A-B are two photomicrographs illustrating cellular expression of an Ad5CMVGFP construct (Figure 9a) and an Ad5PPE-1-GFP construct (Figure 9b) in liver tissue of mice injected with the constructs.
FIG. 10 is a histogram illustrating the effect of introducing an enhancer element according to the present invention into a promoter on levels of expression in endothelial and non-endothelial cell lines.
FIG. 11 is a histogram illustrating the effect of introducing an enhancer element according to the present invention into a promoter on levels of expression in endothelial and non-endothelial cell lines. FIGs. 12A-C are photomicrographs illustrating GFP expression in Ad5PPE-1-3XGFP transduced cells, Ad5PPE-1GFP transduced cells and Ad5CMVGFP transduced cells respectively.
FIGs. 13A-B illustrate GFP expression in SMC transduced by moi-1 of Ad5PPE-1-3XGFP and Ad5CMVGFP respectively.
FIGs. 14A-B show results of an experiment similar to that of Figures 13A-B conducted in HeLa cells.
FIGs. 15A-B show results of an experiment similar to that of Figures 13A-B conducted in HepG2 cells.
FIGs. 16A-B show results of an experiment similar to that of Figures 13a-b conducted in NSF cells.
FIGs. 17A-B are photomicrographs illustrating GFP expression in endothelial cells lining a blood vessel of mice injected with the Ad5PPE-1GFP and the Ad5PPE-1-3XGFP constructs respectively.
FIGs. 18A-C are photomicrographs illustrating results from kidney tissue of injected mice. Ad5CMVGFP injected mice (Figure 18A) , Ad5PPE-1GFP (Figure 18B; slightly higher GFP expression is visible in the blood vessel wall; indicated by arrow) and Ad5PPE-1-3XGFP (Figure 18C).
FIGs. 19A-C illustrate experiments similar to those depicted in Figures 18A-C, conducted on sections of spleen tissue.
FIGs. 20A-D and 20C'-D' illustrate GFP expression in metastatic lungs of control mice injected with Saline (Figure 20A), mice injected with Ad5CMVGFP (Figure 20 B), mice injected with Ad5PPE-1GFP (Figure 20 C) and mice injected with Ad5PPE-1-3XGFP (Figure 20D). Anti Cd31 immunostaining (Figures 20C' to 20D') confirm the co-localization of the GFP expression and CD31 expression in each metastatic tissue.
FIG. 21 is a histogram illustrating that Luciferase activity (light units/µg protein) in BAEC transfected by a plasmid containing the murine PPE-1 promoter is significantly higher when transfected cells were incubated under hypoxic conditions.
FIG. 22 is a histogram as in Figure 21, except that Ad5PPE-1Luc and Ad5CMVLuc were employed.
FIG. 23 is a series of histograms as in Figure 22 showing the effects of hypoxia in different cell lines.
FIG. 24 is a histogram illustrating the effect of the 3X sequence of the present invention on the PPE-1 hypoxia response in BAEC cells. Cells were transduced by Ad5PPE-1Luc and Ad5PPE-1-3XLuc.
FIG. 25 is a histogram showing levels of Luciferase expression in PPE-1-Luc transgenic mice following femoral artery ligation.
FIGs. 26A-B are plasmid maps of constructs employed in conjunction with the present invention.

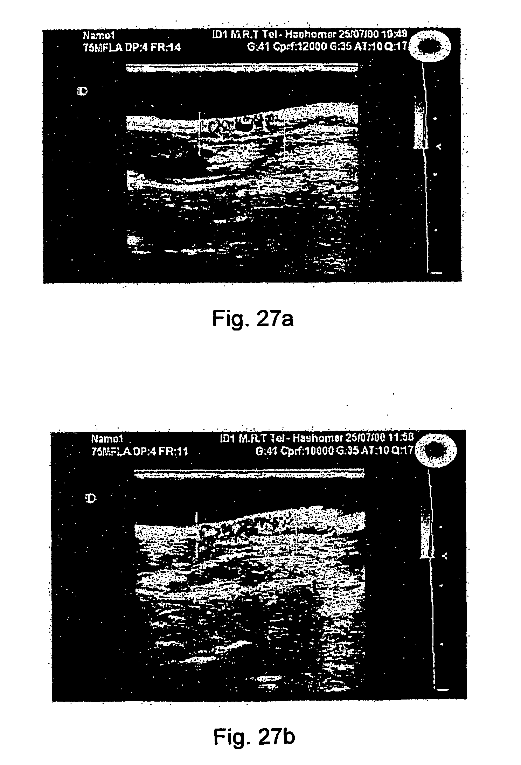

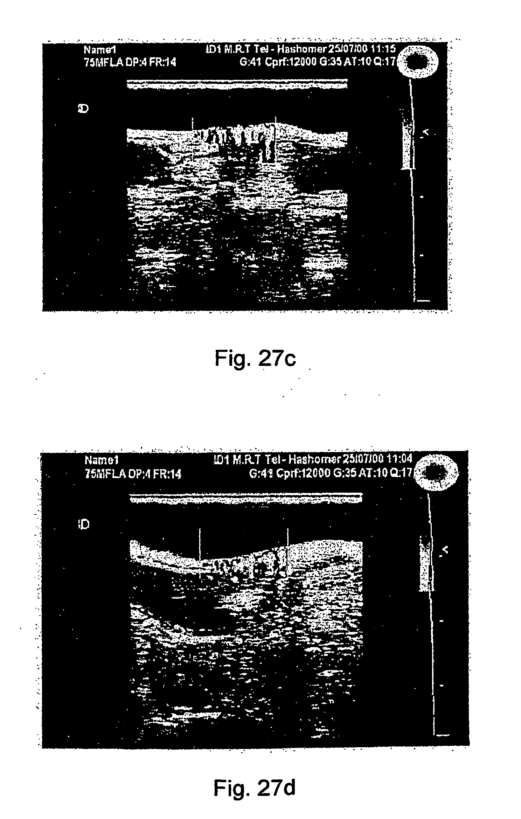

FIGs. 27A-D are a series of ultrasound images of ligated limbs of representative animals from the different treatment groups, 21 days following ligation. Figure 27A Control, Ad5CMVLuc. Treated; Figure 27B Control, saline treated; Figure 27C Ad5PPE-3X-VEGF treated; Figure 27D Ad5CMV-VEGF treated.
FIG. 28 is a histogram illustrating Luciferase activity in proliferating and quiescent Bovine Aortic Endothelial Cells (BAEC) transduced with Ad5PPE-1Luc (open bars) and Ad5CMVLuc (black bars).
FIG. 29 is a histogram illustrating Luciferase activity in BAEC transduced with Ad5PPE-1Luc. during normal proliferation, a quiescent state and rapid proliferation following addition of VEGF.
FIGs. 30A-B are histograms illustrating Luciferase activity (light units/µg protein) in the (Figure 30A) aortas and livers (Figure 30B) of Ad5PPE-1Luc and Ad5CMVLuc normal injected C57BL/6 mice. Activities were determined 1 (n=13), 5 (n=34), 14 (n=32), 30 (n=20) and 90 (n=11) days post injection.
FIGs. 31A-B are histograms illustrating relative Luciferase activity (light units/µg protein) detected five (Figure 31A) and fourteen (Figure 31B) (n=10 for each time point) days post injection of Ad5PPE-1Luc (open bars) or Ad5CMVLuc (black bars) in normal injected BALB/C mice. Activity is expressed as percentage of total body Luciferase expression of each animal.
FIG. 32 is a prior art image depicting an Aorta dissected from ApoE deficient mice colored by Sudan - IV. The thoracic aorta contains less red stained atherosclerotic lesion while the abdominal region includes many red stained atherosclerotic lesions. (Adapted from Imaging of Aortic atherosclerotic lesions by ¹²⁵I-HDL and ¹²⁵I-BSA. A. Shaish et al, Pathobiology - submitted for publication).
FIG. 33 is a histogram illustrating absolute Luciferase activity (light units/µg protein) detected 5 days post systemic injections of Ad5PPE-1Luc (open bars; n=12) or Ad5CMVLuc (black bars; n=12) to ApoE deficient mice. Luciferase activity observed from the abdominal aorta contain high lesion levels and from the thoracic area (low lesion levels).
FIG. 34 is a histogram illustrating absolute Luciferase activity (light units/µg protein) 5 days post systemic injections ofAd5PPE-1Luc (black bars) or Ad5CMVLuc (open bars) to healing wound C57BL/6 induced mice.
FIG. 35 is a histogram illustrating Luciferase activity in normal lung, metastatic lung and primary tumor of Lewis lung carcinoma-induced mice. Lewis lung carcinoma was induced by D122-96 cells injection to the backs for primary tumor model and to the footpad for the metastatic model. Luciferase activity was measured five days post-systemic injection of Ad5PPE-1Luc (n=9; open bars) or Ad5CMVLuc (n=12; black bars). Activity is expressed as light units/µg protein.
FIGs. 36A-D are photomicrographs illustrating GFP expression and tissue morphology in lungs and tumors of LLC bearing mice after intra-tumoral injection of Ad5PPE-1GFP. Tissue was frozen in OCT and sectioned to 10µm by cryostat. All pictures were taken in magnification of 25x. Figure 36A - GFP in angiogenic blood vessels of lung metastases; Figure 36B - CD31 antibody immuno-staining of the section pictured in Figure 36aA; Figure 36C - GFP expression in blood vessels of primary tumor; Figure 36D - phase contrast of the section of C illustrating blood vessels.
FIG. 37 is a histogram illustrating Luciferase expression in normal lung and metastatic lung of Lewis lung carcinoma-induced mice, injected with Ad5CMVLuc, Ad5PPE-1Luc and Ad5PPE-1-3X-Luc Lewis lung carcinoma was induced by D 122-96 cells injected to the foot pad for the metastatic model. Luciferase activity was measured five days post-systemic injection of Ad5CMVLuc (n=7;black bars), Ad5PPE-1Luc (n=6;gray bars), or Ad5PPE-1-3XLuc (n=13;brown bars). Activity is expressed as light units/µg protein.
FIG. 38 is a histogram illustrating Luciferase activity as percentage of liver activity (where the liver is 100%), in normal lung and lung metastasis of Lewis lung carcinoma-induced mice injected with Ad5CMV, Ad5PPE-1Luc and Ad5PPE-1(3X).
FIG. 40 is a histogram illustrating Luciferase activity (light units/µg protein) in muscles (ischemic and normal) of PPE-1Luciferase transgenic mice at two, five, ten and 18 days post femoral ligation and in control (non-ligated animals - day 0; n=8 for each groups).
FIG. 41 is a histogram illustrating Luciferase activity (light units/µg protein) in the liver, lung and aorta in muscles (ischemic and normal) of PPE-1Luciferase transgenic mice at five (n=6), ten (n=6) and 18 (n=8) days post femoral ligation and in control (non ligated animals - day 0).
FIG. 42 is a histogram illustrating Luciferase activity, (light units/µg protein detected in the livers, lungs and primary tumors of LLC mice injected in primary tumors with Ad5CMVLuc (black bars) or Ad5PPE-1Luc (open bars).

### DESCRIPTION OF THE PREFERRED EMBODIMENTS

The present invention is of an improved endothelial cell-specific promoter which can be employed to reliably direct high-level expression of a sequence of interest to endothelial cells and in particular endothelial cells participating in angiogenesis.

The principles and use of the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the examples and drawings. The invention is capable of other embodiments or of being practiced or carried out in various ways. Also, it is to be understood that the phraseology and terminology employed herein is for the purpose of description and should not be regarded as limiting.

Although endothelial specific promoters have been previously described (e.g. United States patent 5,747,340) these promoters have typically been inefficient at directing expression to endothelial cells or have not been demonstrated as being specific to endothelia cells *in-vivo.*

Enhancer elements specific to endothelial cells have also been described. Bu et al. (J.Biol Chem. (1997) 272(19): 32613-32622) have demonstrated that three copies (3X) of the 1X enhancer element of PPE-1 (containing elements ETE-C, ETE-D, and ETE-E) endows promoter sequences with endothelial cell specificity *in-vitro,* however such activity has not been demonstrated *in-vivo.*

As is well known in the art, *in-vitro* experiments cannot reliably predict in-vivo results. As such, the results presented by Bu et al., although suggestive of endothelial cell specificity, do not provide sufficient evidence as to the utility of 3X enhancer element in-vivo.

The lack of in-vivo studies also brings into question the endothelial cell specificity of the 3X enhancer element in whole organisms. Lack of this data implies that therapeutic application of this element is questionable, because when employed *in-vivo,* and in particular when employed for regulating angiogenesis, it is imperative that expression of an angiogenesis regulator (e.g., cell toxin) be directed specifically to endothelial cells, preferably in a specific subset of endothelial cells which are involved in angiogenesis.

As is illustrated in the examples section which follows, the present inventors, through laborious experimentation, have provided, for the first time, conclusive evidence as to the *in-vivo* activity of the 3X enhancer element. Such evidence identifies the 3X element and its sequence derivatives (e.g., SEQ ID NO:7) as highly suitable for use in therapeutic applications.

In addition, in reducing the present invention to practice, it was discovered that a novel configuration of the PPE-1 enhancer sequence of the present invention endows promoter sequences with an unexpected and highly specific activity in endothelial cells participating in angiogenesis.

Thus, according to one aspect of the present invention there is provided an isolated polynucleotide functional as an endothelial cell specific promoter in a mammal such as a human being.

The isolated polynucleotide includes an enhancer element including one or more copies of the sequence set forth in SEQ ID NO:6 and preferably one or more copies of the sequence set forth in SEQ ID NO:8, which as is illustrated in the Examples section which follows, plays an important role in regulating expression in endothelial cells participating in angiogenesis.

One specific and novel sequence configuration of an enhancer element utilizable by the present invention is illustrated in SEQ ID NO:7.

For purposes of this specification and the accompanying claims, the term "enhancer" refers to any polynucleotide sequence which increases the transcriptional efficiency of a promoter.

According to some embodiments of the invention, the isolated polynucleotide includes contiguous copies of SEQ ID NOs:6 and/or 8. Such sequences are preferably positioned in a head-to-tail orientation, although, the enhancer element of the present invention can also include one or more copies of a specific portion of the sequence of SEQ ID NO:6 or 8, in an inverted orientation, e.g., by using sequences complementary to SEQ ID NO:6 or 8 in construction of the enhancer element.

Preferably the isolated polynucleotide further includes an endothelial cell-specific promoter sequence element. For purposes of this specification and the accompanying claims, the term "promoter" refers to any polynucleotide sequence capable of mediating RNA transcription of a downstream sequence of interest. The endothelial specific promoter element may include, for example, at least one copy of the PPE-1 promoter.

Preferably, the isolated polynucleotide further includes a hypoxia response element, for example at least one copy of the sequence set forth in SEQ ID NO: 5.

Thus, according to this aspect of the present invention, an endothelial cell specific promoter which includes various enhancer element configurations is provided.

It will be appreciated that the enhancer element sequences can be positioned within the promoter sequence used, upstream of the promoter, between the promoter and a downstream sequence of interest or within the sequence of interest (e.g., intron).

The isolated nucleic acid sequence of the present invention can be used to regulate gene expression in eukaryotic tissue, and in particular, in proliferating endothelial cells, for example endothelial cells involved in angiogenesis.

Thus, the isolated polynucleotide sequence of the present invention may be provided, in some cases, as part of a nucleic acid construct further including a nucleic acid sequence of interest which is positioned under the regulatory control of the isolated polynucleotide of the present invention. It will be appreciated that such a nucleic acid construct can further include any additional polynucleotide sequences such as for example, sequences encoding selection markers, origin of replication in bacteria, or sequences encoding reporter polypeptides. Such a nucleic acid construct is preferably configured for mammalian cell expression and can be of viral origin. Numerous examples of nucleic acid constructs suitable for mammalian expression are known in the art; the Examples section which follows provides further detail of several such constructs.

For purposes of this specification and the accompanying claims, the phrase "sequence of interest" refers to any polynucleotide sequence which has the capacity to be transcribed by an RNA polymerase. This definition includes coding sequences translatable into polypeptides, as well as sequence for antisense RNA, RNA which binds DNA, ribozymes and other molecular moieties which are not destined to undergo translation. Examples of nucleic acid sequence of interest which may be used by the construct according to the present invention are provided hereinbelow and in the Examples section which follows.

Examples presented hereinbelow illustrate that the improved endothelial cell specific promoters of the present invention can reliably direct expression of a reporter gene to endothelial tissue after systemic in-vivo administration. These examples further show, for the first time, that the isolated polynucleotide of the present invention can be used to preferentially express a reporter protein (GFP) in atherosclerotic and/or angiogenic tissue, thus providing for the first time direct evidence as to the importance of the PPE-1 enhancer element and its derivative in therapeutic applications.

While use of a reporter protein, such as GFP, may have utility in detection of early stages of metastatic tumor growth, especially in animal models, or for non-invasive imaging of metastases (Yang, M. et al., Proc. Nat. Acad. of Sci. (2001) 27:2616-2621) such a use is only a small portion of the projected utility of the claimed invention. It is believed, for example, that AdPPE-1GFP can be used in a combination with AdPPE1tk, AdPPE-1p55 and/or other anti-angiogenic treatments, in order to follow and treat angiogenesis by a relatively non-invasive method.

Replacement of the GFP reporter gene with an apoptosis inducing factor (e.g. p55; GenBank accession M75866) in a construct of, for example AdPPE-1-3X-p55 is predicted to reliably target apoptosis to rapidly proliferating endothelial cells in angiogenic blood vessels of a growing tumor. Because such a vector may be administered systemically, it can be employed to effectively induce apoptosis in developing metastatic foci, without discovering the location of those foci. Such a use represents a significant improvement in comparison to prior art practice. By inducing apoptosis specifically in developing vasculature, it is feasible to eliminate angiogenesis.

An opposite approach may be used to re-vascularize tissue, for example in atherosclerotic patients or in patients that have suffered significant impairment of peripheral circulation as a result of disease or injury. In this case, a construct of the type AdPPE-1-3X-GF, where GF is a growth factor (e.g., cytokine) or modificants thereof (e.g., AdPPE-1-SEQ ID NO:7-GF), can be employed. Suitable growth factors for use in this context include, but are not limited to, VEGF (GenBank accession M95200) and rat PDGF- BB (GenBank accession; 99% identity to mus-AF162784) and EGR-1 (GenBank accession M22326) FGFs (including, but not limited to, GenBank accession XM 003306) and combinations thereof.

It will be appreciated that incorporation of a hypoxia response element (e.g. SEQ ID NO: 5) within the promoter sequence of the present invention can be used to further enhance expression selectivity to Ischemic tissues, thus leading to neo-vascularization of selected tissues. As the blood supply improves, Ischemia is relieved, the hypoxia response element ceases to be induced, GF levels decline and the neo-vascularization process is halted.

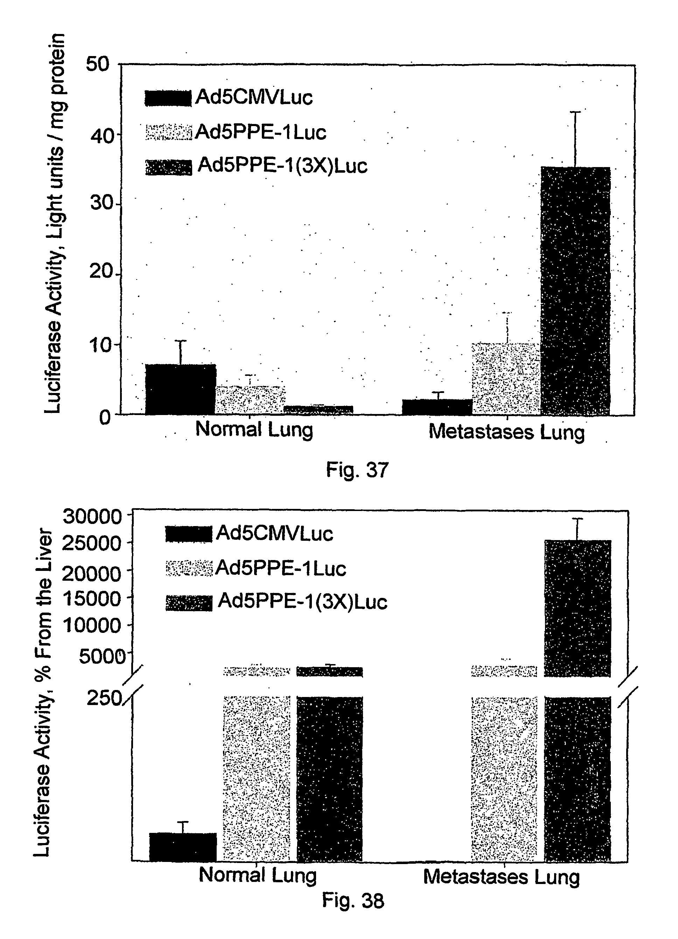

The promoter sequences generated according to the teachings of the present invention are particularly useful in regulating angiogenesis in a tissue. As illustrated in the Examples section which follows, the modified 3X (SEQ. ID. NO:7) containing promoter sequence of the present invention and the unmodified PPE-1 promoter are both expressed in metastatic foci of the LLC model. However example 22 clearly illustrates that the modified 3X sequence is specifically responsible for both a decrease in expression levels of the reporter gene in normal lung and a dramatic increase in expression of the reporter gene in metastatic foci. There is neither a hint nor a suggestion in the prior art that such a result could be achieved. Thus, use of a construct including the 3X element in a gene therapy context can be expected to maximize delivery to tumors while minimizing toxic effects on surrounding normal tissue. Significantly, this is true even if the surrounding tissue contains an endothelial component, as illustrated in Figure 37. This is because, as demonstrated in example 11, the 3X sequence greatly increases the level of expression in rapidly proliferating endothelial tissue, even in the context of the PPE-1 promoter.

For example, the p55 gene might be used in conjunction with a promoter of the present invention containing a hypoxia response element in order to specifically induce apoptosis in growing tumors. Such a strategy is deemed feasible because a growing tumor mass tends toward ischemia as tumor growth often exceeds the angiogenic capacity of the surrounding tissue. Other expressible cell toxins which can be used along with the promoter sequence of the present invention in order to specifically reduce a tumor mass include but are not limited to, other pro-apoptotic genes, the Herpes simplex thymidine kinase gene (HSV-tk; included in the pORF-HSV1tk expression vector available from InvivoGen, San Diego, CA) angiostatin (Genbank accession number X05199), endostatin (Genbank accession number M33272) and angiostatin-endostatin chimera (included in the pORF-HSV1tk expression vector available from InvivoGen, San Diego, CA).

Alternately, or additionally, angiostatin or endostatin genes might be used in conjunction with a promoter of the present invention in order to specifically block angiogenesis without inducing apoptosis.

Thus, according to alternate preferred embodiments, angiogenesis may be stimulated or blocked. This flexibility will allow varied uses of the invention including, but not limited to reduction of tumor mass and revascularization of atherosclerotic regions of the heart or neo-vascularization of peripheral tissues with an inadequate blood supply. One relevant clinical scenario is use of a promoter according to the present invention to generate new blood vessels to increase the blood supply in limbs of diabetic patients.

The nucleic acid construct according to the present invention can be administered to a subject (mammals, preferably humans) *per se,* or in a pharmaceutical composition where it is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the nucleic acid construct accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

It will be appreciated that the isolated polynucleotide of the present invention has been isolated based on its capacity to promote or enhance transcription in eukaryotic cells of an endothelial lineage. Therefore a mammalian cell transformed with a claimed isolated polynucleotide is an additional embodiment of the invention. Numerous examples of such transformed cells are provided in examples recited herein below.

While the examples provided hereinbelow deal specifically with the use of the 3X sequence in conjunction with the PPE-1 promoter, it is anticipated that the enhancer sequence of the present invention will also exert its cell specific effect when used with other eukaryotic promoter sequences.

Such anticipation is based on prior art findings which show that enhancer elements are often portable, i.e., they can be transferred from one promoter sequence to another, unrelated, promoter sequence and still maintain activity. For examples, see D. Jones et al. (Dev. Biol. (1995) 171(1):60-72); N. S. Yew et al, (Mol. Ther. (2001) 4:75-820) and L. Wu. et al. (Gene Ther. (2001) 8;1416-26). Indeed, the earlier work of Bu et al. (J.Biol Chem. (1997) 272(19): 32613-32622) strongly suggests that enhancer elements related to those of the present invention, for example enhancers including SEQ ID NO: 6 may be used with constitutive promoters, for example the SV-40 promoter. As such, constructs containing, methods employing and isolated polynucleotides including a eukaryotic promoter modified to include the enhancer sequence of the present invention are well within the scope of the claimed invention.

Thus, it is postulated that a minimal configuration of an enhancer element according to the present invention is an isolated polynucleotide as set forth in SEQ ID NO:8. This enhancer is anticipated to function with a wide variety of promoters, including but not limited to endothelial specific promoters (e.g. PPE-1; SEQ ID NO.: 1) and constitutive promoters, for example viral promoters such as those derived from CMV and SV-40. This enhancer should be capable of imparting endothelial specificity to a wide variety of promoters. The enhancer element may be augmented, for example by addition of one or more copies of the sequence set forth in SEQ ID NO:6. These additional sequences may be added contiguously or non-contiguously to the sequence of SEQ ID NO.: 8.

The present invention further includes a method of expressing a nucleic acid sequence of interest in endothelial cells employing a construct which relies upon an enhancer element including at least one copy of the sequence set forth in SEQ ID NO:8 and a promoter to direct high level expression of the sequence of interest specifically to endothelial cells.

As used herein "ex-vivo administration to cells removed from a body of a subject and subsequent reintroduction of the cells into the body of the subject" specifically includes use of non-human stem cells as described in (Lyden et al. (2001) Nature Medicine 7:1194-1201).

While adenoviruses are employed in the experiments described in examples presented hereinbelow, the constructs of the present invention could be easily adapted by those of ordinary skill in the art to other viral delivery systems.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non-limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); Nucleic Acid Hybridization" Hames, B. D., and Higgins S. J., eds. (1985); "Transcription and Translation" Hames, B. D., and Higgins S. J., eds. (1984); "Animal Cell Culture" Freshney, R I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996).

Other general references are provided throughout this document. The procedures therein are believed to be well known in the art and are provided for the convenience of the reader.

Specifically, experiments conducted in conjunction with the examples recited hereinbelow employed the following methods and materials:

### Materials and Methods

### Cell culture

Lewis Lung Carcinoma - (D122-96) (kindly provided by Prof. L. Eisenbach, The Weizmann Institute of Science Rehovot, Israel), Human Embryonic Kidney (293) and HeLa cells were grown in 4.5gr/l DMEM, supplemented with 10% fetal calf serum (FCS), 50 U/ml penicillin, 50 µg/ml streptomycine and 2 mM glutamine (Biological industries, Beit-Haemek, Israel). Bovine Aortic Endothelial Cells - BAEC (kindly provided by Prof. N. Savion, Goldshlager Institute, Sheba Medical Center, Tel-Hashomer, Israel), Normal Skin Fibroblasts - NSF, HepG2 and Human Umbilical Endothelial Cells - HUVEC-304 (ATCC, USA) were grown in 1.0gr/l DMEM (Biological industries, Beit-Haemek, Israel), supplemented with 5% FCS, 50 U/ml penicillin, 50 µg/ml streptomycine and 2 mM glutamine. The BAEC cells were supplemented with complete fibroblast growth factor (Sigma, St. Louis. MO.). RINr1046-38 (RIN-38) were grown in 199 Earle's salts (5.5M glucose) medium supplemented with 5% FCS (Biological Industries, Beit-Haemek, Israel), 50U penicillin/ml, 50 µg streptomycine/ml and 2 mM glutamine.
"HepG2" as used herein refers to ATCC-HB-8065.
"HeLa" as used herein refers to ATCC-CCL-2.
"Human Bronchial Epithelial cells" and "B2B" as used herein refers to ATCC-CRL-9609.
"HUVEC" and "Human Umbilical Vein Endothelial Cells" as used herein refers to ATCC-CRL-1730.
"CHO" and "Chinese Hamster Ovary" as used herein refers to ATCC- 61.

### Hypoxia Induction

Twenty six hours post transfection or transduction cells were incubated in an isolated chamber which was washed for 30 minutes by a gas flow containing 0.5%O₂, 5%CO₂ balance by N₂. The isolated chamber was placed in humidified 5% CO₂ 37°C incubator.

### Luciferase activity in cells and tissues

To assay the PPE-1 promoter activity quantitatively *in-vitro* and *in-vivo,* a Luciferase gene expression system kit was employed (Promega Corp., Madison, WI). Forty eight hours post transfection or transduction the cells were washed and 200µl lysis buffer was added for 15 minutes. Cells lysates were collected and centrifuged for 15 minutes (14,000rpm) at 4°C. Subsequently, 10µl of the supernatant was added to 50µl Luciferase assay buffer. The activity was measured in Luminometer over a 20 second period.

To assay Luciferase activity in solid tissue a 20mg sample was excised and homogenized in 1ml of the homogenization solution and centrifuged for 15 minutes (14,000rpm) at 4°C, and 10 ml of the supernatant were assayed for Luciferase activity, as described above. Results were expressed as Luciferase light units per 1µg protein. Protein was measured using the Bradford assay with bovine serum albumin (BSA) as a standard.

### GFP activity in-vitro and in-vivo

To test GFP expression in-vitro, cells were washed twice with PBS and were fixed for 30 minutes with freshly made 4% paraformaldehyde in PBS. After fixation, examination by fluorescent microscopy was conducted.

In order to test the cellular distribution of the delivered gene *in-vivo,* tissues were fixed in freshly made 4% paraformaldehyde in 0.1 M phosphate buffer for 6 hours at 4°C, soaked overnight in 30% sucrose at 4°C and frozen in OCT compound (Sakura, USA). The tissue blocks were sliced by a cryostat at 10 µm thickness and observed directly under fluorescence microscopy (FITC filter).

### Proliferating and quiescent cells

In order to compare the PPE-1 promoter activity in proliferating and quiescent BAEC, the cells were divided into two groups: 1. proliferating cells - growing and infecting in 10% FCS media. 2. quiescent cells - growing and infected in serum free media started in 72 hours prior to the transduction.
All cells were grown in humidified incubator, 5% CO₂, 37°C.

### Preparation of recombinant replication deficient adenoviruses.

Several recombinant replication deficient adenoviruses (type 5) were constructed. An expression cassette including the murine preproendothelin-1 (PPE-1) promoter (SEQ ID NO:1) located upstream to the Luciferase gene (originated from pGL2-basic GenBank Accession number X65323) and the SV40 polyA site (originated from pGL2-basic GenBank Accession number X65323) was ligated into the BamHI restriction site of pPAC.plpA (promoterless construct). The GFP gene (originated from pEGFP, GenBank accession number AAB02572) was ligated to the PPE-1 promoter at the NotI restriction site. The replication deficient recombinant adenoviruses termed Ad5PPE-1Luc or Ad5PPE-1GFP were prepared by co-transfection of pPACPPE-1Luc or Ad5PPE-1GFP with adenovirus plasmid pJM17 as described by Becker, T.C. et al. (Methods Cell biol. 43, Roth M. (ed). New York. Academic Press, 1994, pp. 161-189) followed by harvest of recombinant virions.

Viruses were prepared for large-scale production. The viral stocks were stored at 4°C at concentration of 10⁹-10¹² plaque-forming units/ml (pfu/ml). The viruses Ad5CMV-Luc (kindly provided by R. Gerard from UTSw Dallas, Texas) and Ad5CMV-GFP (Quantum biotechnologies, Carlsbad, Canada) containing the cytomegalovirus (CMV) immediate early promoter (GenBank Accession number U47119) were prepared for large scale preparation as described for the PPE-1 viral vectors and were used as a non-tissue specific control.

### Modfications of the PPE promoter

The modified murine PPE-1 promoter was developed by inserting three copies of the positive transcription element discovered by Bu et al (J.Biol Chem. (1997) 272(19): 32613-32622) into the NheI restriction enzyme site located downstream (-286bp) to the 43 base pairs endogenous positive element (-364 to -320 bp).

The enhancer fragment termed herein "3X" is a triplicate copy of an endogenous sequence element (nucleotide coordinates 407-452 of SEQ ID NO:1) present in the murine PPE-1 promoter. It has been previously shown that induction of PPE-1 promoter activity in vascular endothelial cells depends on the presence of this element Bu et al (J.Biol Chem. (1997) 272(19): 32613-32622). The 3X fragment was synthesized by using two complementary single stranded DNA strands 96 base pares in length (BioTechnology industries; Nes Tziona, Israel), (SEQ ID NO: 2 and 3). The two single stranded DNA fragment were annealed and filled using Klenow fragment (NEB); the resulting double stranded DNA was 145 base pairs long and included Nhe-1 restriction sites (SEQ ID NO: 4).

The 3X fragment was ligated into the murine PPE-1 promoter down stream of endogenous Nhe-1 site using T4 Ligase. The resulting construct was propagated in DH5α compatent cells and a large-scale plasmid preparation was produced using the maxi-prep Qiagene kit.

### Additional Plasmids

### wild type PPE-1 promoter

The PPE-1-Luciferase cassette (5249bp) containing 1.4kb of the murine preproendothelin-1 (PPE-1) promoter, the Luciferase gene with an SV40 polyA signal (GenBank Accession number X 65323) site and the first intron of the murine ET-1 gene is originated from the pEL8 plasmid (8848bp) used by Harats et al (J. Clin. Inv. (1995) 95: 1335-1344). The PPE-1-Luciferase cassette was extracted from the pEL8 plasmid by using the BamHI restriction enzyme, following by extraction of the DNA fragment from a 1% agarose gel using an extraction kit (Qiagen, Hilden, Germany).

### The promoter-less pPAC.plpA plasmid

The promoter-less pPAC.plpA plasmid (7594bp) containing sequences of the adenovirus type 5 was originated from the pPACCMV.pLpA (8800bp). The CMV promoter, the multiple cloning site and the SV40 polyadenylation site (1206 bp) were eliminated by NotI restriction enzyme, The fragmented DNA was extracted from 1% agarose gel. The linear plasmid (7594 bp) was filled-in by Klenow fragment and BamHI linker was ligated by rapid DNA ligation kit to both cohesive ends. The linear plasmid was re-ligated by T4 DNA ligase and transformed into DH5α competent cells, in order to amplify the pPAC.plpA with the BamH1 restriction sites. The plasmid was prepared for large-scale preparation and purified by maxi prep DNA purification kit.

### pPACPPE-1Luciferase plasmid

The pPACPPE-1Luciferase plasmid was constructed by inserting the PPE-1-Luciferase cassette into the BamHI restriction site of the pPAC.plpA plasmid, by using T4 DNA ligase. The plasmid was subsequently used to transform DH5α competent cells. The plasmid (12843bp) was prepared for large-scale preparation and purified by maxi prep DNA purification kit.

### pPACPPE-1GFP plasmid

The pPACPPE-1GFP plasmid was constructed by sub-cloning the GFP gene (originated from pEGFP, GenBank accession number AAB02572) downstream to the PPE-1 promoter into the NotI restriction site, by T4 DNA ligase.

The plasmid was subsequently used to transform DH5α competent cells. The plasmid (11,801 bp) was prepared for large-scale preparation and purified by maxi prep DNA purification kit.

### pACPPE-13X Luciferase and pACPPE-13X GFP plasmids

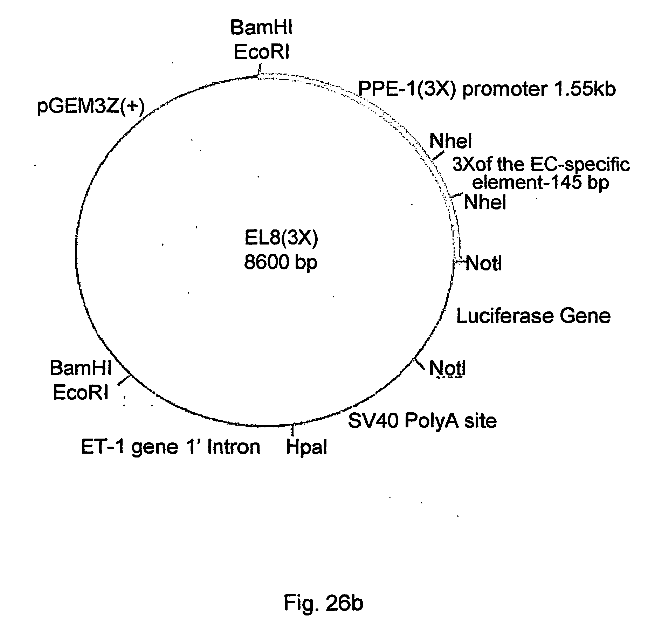

The pPACPPE-1-3XLuciferase and pPACPPE-1-3XGFP were constructed by inserting the PPE-1-3XLuc or PPE-1-3XGFP cassette digested by BamHI restriction enzyme from pEL8-3X (Figure 26B) containing Luc or GFP into the BamHI restriction site of the pPAC.plpA plasmid. pEL8-3X contains the modified murine PPE-1 promoter (1.55kb) (red) - located between BamHI and NotI that contains the triplicate endothelial specific enhancer 3X (as set forth in SEQ ID NO.: 7) located between two NheI site. The promoter, the Luciferase or GFP gene, the SV40 poly A sites and the first intron of the endothelin-1 gene, all termed the PPE-1 modified promoter cassette was digested and extracted by BamHI restriction enzyme as described in materials and methods. The plasmids (12843bp) were prepared for large-scale preparation and purified by maxi prep DNA purification kit.

### In-vitro experiment, DNA transduction

Cells were plated in 16 mm dishes 24 hours before transduction. DNA transduction of BAEC (Bovine Aortic Endothelial Cells), HUVEC (Human Umbilical Vein Endothelial Cells), LLC (Lewis Lung Carcinoma) and RIN (Rat insulinoma), HepG2, HeLa and Normal skin fibroblasts (NSF) cells was performed by incubating each cell line with 1, 5 and 10 multiplicity of infection (moi) of Ad5PPE-1Luc for 4 h in a total volume of 500 µl growing media, following by incubation with the growing media in a total volume of 2 ml for 48 hours. Ad5CMVLuc was used as a non-tissue specific control.

### Animals

All animal procedures were approved by the "Animal Care and Use Committee" of Sheba Medical Center, Tel-Hashomer.

### Different mouse strains were used:

(i) Male, 3 months old, wild type C57BL/6 mice (Harlan farms, Jerusalem, Israel).
(ii) Male 3 month old BALB/C mice (Harlan farms, Jerusalem, Israel).
(iii) Male and female 6 month old ApoE gene deficient mice hybrids of C57BL/6xSJ129 mice (Plump AS. et al. Cell (1991) 71:343-353).
(iv) Male and female 3 month old over-expressing the Luciferase gene under the control of murine PPE-1 promoter (5.9Kb), generated by Harats et al. (J. Clin. Inv. (1995) 95: 1335-1344).

All mice were grown in the Lipids and Atherosclerosis Research Institute.

### Tissue, gene expression in normal mice

To assay the efficiency and tissue specificity, 10¹⁰ pfu/ml of Ad5PPE1Luc or Ad5CMVLuc (as non-tissue-specific control), were suspended in 100 µl of physiological saline and injected into the tail vein of mice as described hereinabove. Luciferase activity was assayed 1, 5, 14, 30 and 90 days post-injection. To localize cellular distribution of the expressed reporter genes, Ad5PPE-1GFP or Ad5CMVOFP (10¹⁰ pfu/ml in 100 µl physiological saline) were injected into the tail vein of normal 3 month old, male C57BL/6 mice. GFP expression was detected five days post-injection. All mice appeared healthy and no toxicity or inflammation was noted in the liver or other tissue.

### GFP activity in tissues

To test the cellular distribution of the delivered gene in-vivo, tissue samples from injected mice were fixed in freshly made 4% paraformaldehyde in 0.1 M phosphate buffer for 6 hours at 4°C, soaked overnight in 30% sucrose at 4°C and frozen in OCT compound (Sakura, California, USA). The tissue blocks were sliced at 10 µm thickness and observed directly under fluorescence microscopy (FITC filter).

### Tumor implantation

Lewis Lung Carcinoma cells (LLC) were harvested with trypsin/EDTA, washed 3 times with PBS and counted with 0.1% trypan blue (Biological industries, Beit-Haemek, Israel) to assess their viability. In order to test the level of activity of the PPE-1 promoter activity in tumor angiogenesis in mice, two different tumor models were used.

In the primary tumor model, the cells (1x10⁶ cells/ml in 100 µl physiological saline) were subcutaneously injected to the mice backs (n=17). Twenty-one days post injection Ad5PPE-1, Ad5PPE-1GFP, Ad5CMV, or Ad5CMVGFP (10¹⁰pfu/ml) were injected into the tumor tissue (IT) or intravenously and their activity was detected as described above.

In the metastatic tumor model, the cells (5x10⁵ cells/ml in 50 µl physiological saline) were injected to the mice foot-pad (n=12). When the tumor tissue reached a size of 0.7 mm in diameter, the foot pad (with the primary tumor) was resected under anaesthetic and sterile conditions. Fourteen days post surgery the viruses (Ad5PPE-1, Ad5PPE-1GFP, Ad5CMVLuc or Ad5CMVGFP) were injected to the mouse tail vein.

In both tumor experimental models mice were sacrificed 5 days post viral injection, their tissues were excised and tested for Luciferase or GFP activities.

### Wound healing model

Male 3 month old C57BL/6 mice were anaesthetized by subcutaneous injection of sodium pentobarbital (6mg/kg). Their backs were shaved and 5 cm of straight incisions was made. The incisions were immediately sutured by 4/0 sterile silk thread. The angiogenic process in the healing wound was examined every two days by H&E and anti von-Willibrand antibody immuno-histochemistry staining.

Ten days post incisions 10¹⁰pfu/ml of Ad5PPE-1Luc or Ad5CMVLuc were systemically injected to the tail vein. Five days post injections the mice were sacrificed and Luciferase activity was assayed as described above in the skin of the incision site and in the normal contra lateral site as a control.

### Histological examination

In order to evaluate the extent of angiogenesis in tumor and metastasized tissue, the tissues were sliced into 5 µm sections and stained with Haematoxylin and Eosin (H&E). Anti CD31 (rat anti mouse CD31 monoclonal Ab. Pharminogen, NJ, USA) antibodies were used for analyses of neo-vascularization in the tumor models.

### Statistical analysis

Analysis between groups for statistically significant differences was performed with the use of t-test ANOVA, or the Mann-Whitney Rank test. Data are shown as mean+SE.

### EXAMPLE 1

### Analysis of 3X-PPE-1 plasmid activity in-vitro

In order to analyze the activity of the PPE-1-3X, a comparison of reporter gene expression in the PPE-1-3X promoter plasmid and the unmodified PPE-1 promoter plasmid was undertaken. Reporter gene plasmids containing either the PPE-1-3X fragment or the unmodified PPE-1 fragment and the reporter gene Luciferase were transfected into endothelial and non-endothelial cell lines as well as to a bronchial epithelium cell line (B2B) which express the PPE-1 promoter (see materials and methods above). The B2B cell line was chosen to provide an indication of the 3X element's capacity to reduce expression in non-endothelial cell lines relative to the PPE-1 promoter. Transfection was accomplished using lipofectamine (Promega Corp., Madison, WI). A βgal-neo plasmid was employed as an indicator of the transfection efficiency in each case according to accepted molecular biology practice.

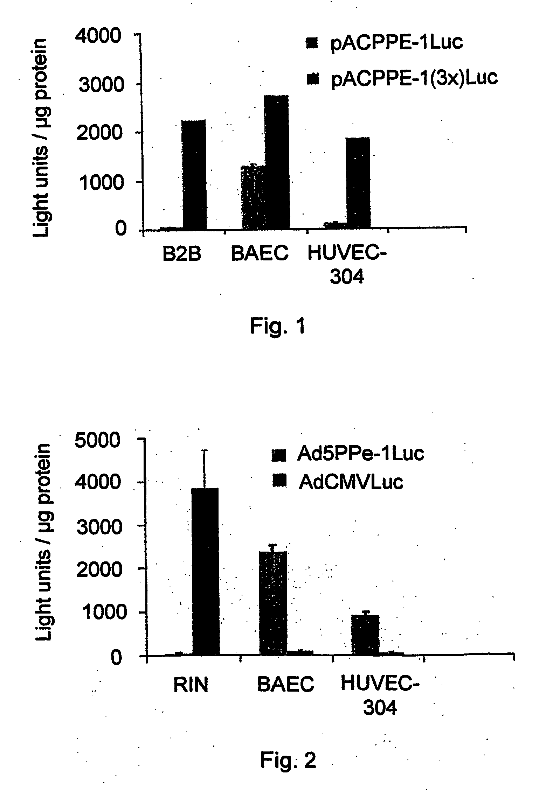

Forty-eight hours post transfection, the cells were harvested using lysis buffer (Promega Corp., Madison, WI) and Luciferase activity was analyzed by a luminometer (TD-20e - Turner Designs, Sunnyvale, California). In parallel, βgal activity was analyzed in order to standardize for different transformation efficiencies. The results are summarized in Figure 1 and Table 1. Luciferase activity under the control of PPE-3X is 15-20 times higher than Luciferase activity under the control of the unmodified PPE-1. In non-endothelial cell lines minimal expression was detected using both the PPE-1 and PPE-1-3X. This demonstrates that PPE-3X is a promising candidate for delivery of a gene specifically into endothelial cells *in-vivo.*

**Table 1 - Luciferase activity in cells transfected with PPE-1 and PPE-1-3X Luciferase constructs**

| Plasmid | Luciferase activity in: endothelial cell lines | | non endothelial cell lines |
|---|---|---|---|
| | HUVAC | BAEC | RIN |
| PPE-1 | 135.12 | 1121.3 | 0.73 |
| PPE-1-3X | 768 | 18331.7 | 0.32 |

### EXAMPIE 2

### Activity and specificity of Ad5PPE-1/Luciferase in-vitro

The PPE-1/Luciferase, PPE-1-3X/Luciferase, PPE-1/GFP and PPE-1-3X/GFP were also ligated into the Ad5 plasmid to produce AdSPPE-1/Luc and Ad5PPE-1-3X/luc, Ad5PPE-1/GFP and Ad5PPE-1-3X/GFP (Varda-Bloom et al., (2001) Gene therapy 8:819-827). These constructs were assayed separately as detailed hereinbelow.

In order to test the activity of the Ad5PPE-1/luc, transfections of B2B (Human bronchial epithelial), BAEC (Bovine Aortic Endothelial Cells) and HUVEC (Human Umbilical Vein Endothelial Cells) were undertaken. These three cell lines express the endothelin gene and were chosen to indicate levels of expression of the tested construct in an endothelial cell. The RIN (Rat Insulinoma) cell line , which does not express endothelin, was employed as a negative control and transfected with the same construct. Ad5CMVLuc (Luciferase under the control of CMV promoter) was used as non-endothelial-specific control in all cell lines.

Figure 2 clearly illustrates that higher Luciferase expression was achieved in endothelial BAEC and HUVEC cell lines with the PPE-1 promoter than with the CMV promoter. In the RIN cells, which are not of endothelial origin, the CMV promoter produced more Luciferase activity than the PPE-1 promoter. These results demonstrate the endothelial specificity of the unmodified PPE-1 promoter.

### EXAMPLE 3

### Activity and specificity of Ad5PPE-3XLuc and Ad5PPE-3XGFP

The Ad5PPE-3X/Luciferase and Ad5PPE-3X/GFP constructs were used to transfect the cell lines described hereinabove in Example 2 in order to ascertain the impact of the 3X element on specificity and expression levels. As in example 2, Ad5CMVLuc was used, as a non-endothelial-specific control. Higher Luciferase expression in BAEC and HUVEC cell lines was detected under the control of the PPE-3X promoter as compared to the CMV promoter.

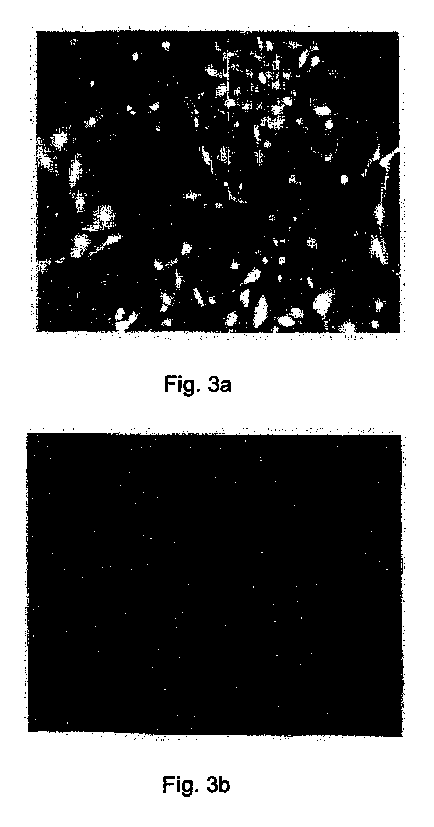

Figure 3a is a photomicrograph illustrating GFP expression under the control of Ad5PPE-1-3X in the BAEC cell line. Figure 3b is a photomicrograph illustrating GFP expression of Ad5CMV in the BAEC line. As is clearly shown by these Figures, the PPE-1-3X promoter is more active in endothelial cells. These results clearly indicate that the 3X element does not detract from the endothelial specificity of the PPE-1 promoter. Relative activities of the PPE-1 and PPE-1-3X promoters in cell culture are presented in example 6 hereinbelow.

### EXAMPLE 4

### In-vitro assay of pro-apoptotic activity of the p55 gene

Following sub cloning of P55 (TNFR1, GenBank accession number M75866) into PACPPE3X (containing the PPE-1-3X promoter), and into PACCMV, co-transfection of these plasmids and GFP (pEGFP-C1 vector; CLONTECH, Palo Alto, CA). was performed as described hereinabove. Briefly, the gene was subcloned downstream to the PPE-1 promoter (instead of the luciferase gene) into the NotI restriction site, by T4 DNA ligase, following by transforming it into DH5α competent cells. Twenty four hours post-transfection, small and rounded apoptotic cells were visually discernible from normal cells. Electron microscopy of cells transfected with the pro-apoptotic plasmids showed typical appearance of apoptosis, confirming the visual evaluation.

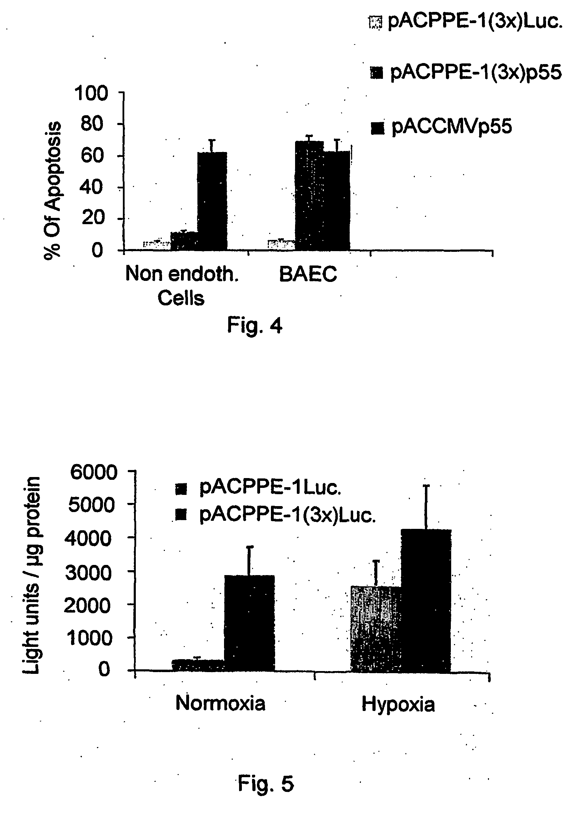

Under the control of the PPE-1-3X promoter, apoptosis was induced by p55 only in endothelial cells (Figure 4), whereas the CMV promoter did not show any cell specific activity. Luciferase under the control of PPE-1-3X did not induce apoptosis in any tested cell lines. These results indicate that by employing the PPE-1-3X promoter, it is feasible to induce apoptosis specifically in endothelial cells.

### EXAMPLE 5

### Hypoxia responsive element (HRE) can enhance target gene expression in hypoxic sensitive endothelial cells

Hypoxia is an important regulator of blood vessels' tone and structure. It has also been shown to be a potent stimulus of angiogenesis (in both ischemic heart diseases and cancer (Semenza, G.L. et al. (2000) Adv Exp Med Biol.; 475:123-30; Williams, K.J. (2001) Breast Cancer Res. 2001: 3;328-31 and Shimo, T. (2001) Cancer Lett. 174;57-64). Further, hypoxia has been reported to regulate the expression of many genes including erythropoietin, VEGF, glycolytic enzymes and ET-1. These genes are controlled by a common oxygen-sensing pathway, an inducible transcription complex termed hypoxia inducible factor-1 (HIF-1). The HIF-1 complex mediates transcriptional responses to hypoxia by binding the cis acting hypoxia responsive element (HRE) of target genes. The HRE is a conserved sequence located in the promoters of few genes that respond to hypoxia including: VEGF, Nitric Oxide Syntase-2, erytropoietin and others including endothelin-1, ET-1. The ET-1 promoter contains an inverted hypoxia response element at position -118 bp upstream of the transcription start site, the element contain 7 base pairs and is located between the GATA-2 and AP1 sites 5' GCACGTT 3' - 50 base-pairs. (SEQ ID NO: 5.)

The preproendothelin-1 (PPE-1) promoter contains an hypoxia responsive element (HRE) that has the potential to increase its expression in the hypoxic microenviroment of tumor or ischemic tissues, thus making it "tumoral tissue specific" and/or "ischemic tissue specific". In order evaluate the actual function of this HRE, assays of the PPE-1 promoter and PPE-1-3X promoter in conjunction with a Luciferase or GFP reporter gene and delivered by an adenoviral vector were undertaken.

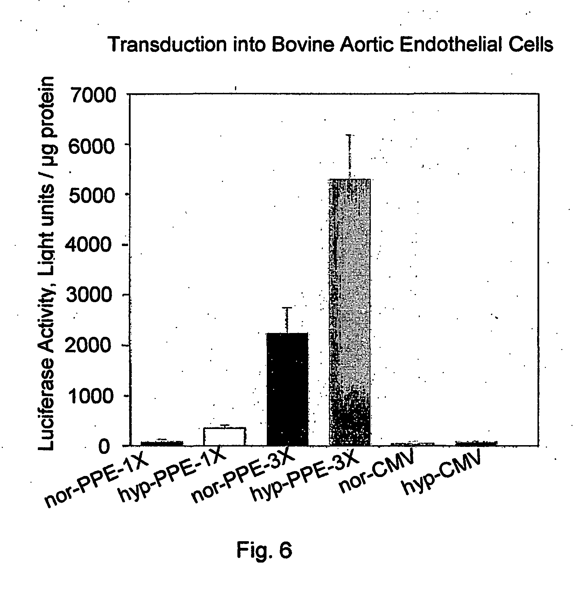

Luciferase activity under the control of the PPE-1 promoter or the PPE-1-3X promoter was compared in BAEC cells under normoxic and hypoxic conditions (0.5% O₂ for 16h). The Luciferase activity under the control of PPE-1 promoter was 5 times higher when exposed to hypoxia (Figures 5 and 6). Further, the Luciferase activity under the control of PPE-1-3X promoter was 2.5 times higher under hypoxic conditions. In summary, introduction of the 3X element into the PPE 1 promoter is till capable of increasing expression levels of a downstream gene in response to hypoxia, even though the normoxic levels of expression with the PPE-1-3X gene are higher than those observed with the unmodified PPE-1 promoter.

### EXAMPLE 6

### Further evaluation of PPE-1-3X and PPE-1 promoter activity in endothelial cell lines

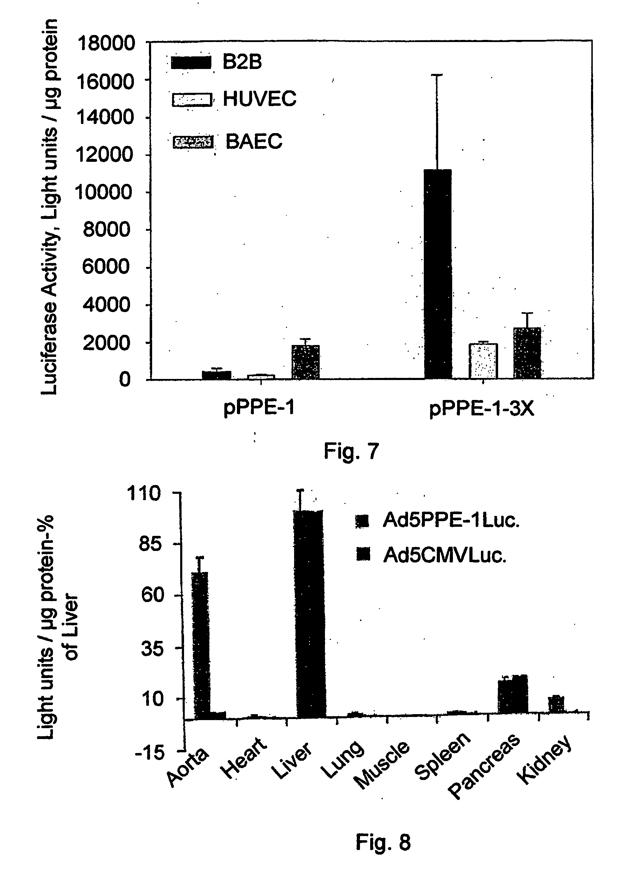

Figure 7 summarizes the results from B2B, HCTVEC and BAEC transfection experiments using pPPE-1/Luciferase and pPPE-1-3X/Luciferase. Higher Luciferase expression (30, 8.5 and 1.5 times more) was observed under the control of the PPE-1-3X promoter than under the PPE-1 promoter in B2B, HUVEC and BAEC, respectively. These results confirm those presented hereinabove and serve to establish that PPE-1-3X is well suited to directing high level expression specifically to endothelial cells. In the context of future in-vivo delivery, the higher levels of expression achieved with the PPE-1-3X construct translate into administration of smaller amounts of DNA. This, in turn, will serve to increase specificity even further.

### EXAMPLE 7

### Efficiency, specificity and stability of Ad5PPE-1Luc in-vivo

In order to confirm that the endothelial specificity of expression observed in examples 2 through 6 was not an artifact of cell culture, the Ad5PPE-1/Luciferase construct was injected into C57BL/6 mice as described hereinabove in "Tissue gene expression in normal mice". As in the in-vitro studies, Ad5CMWLuciferase was employed as a negative control.

Following injection of adenoviral vectors, the specific activity and stability of Luciferase in vascularized and non-vascularized tissues was assayed. Results are summarized in Figure 8 (Luciferase expression relative to expression in liver) and Table 2 (Luciferase expression as a percentage of total expression in the body). As expected, in Ad5CMV/Luciferase treated mice most of the Luciferase activity (>80% of the total body expression) was found in the liver. Luciferase activity controlled by the PPE-1 promoter was lower in the liver (37-54% of the total body expression). The PPE-1 derived expression was much higher in the aorta (23-33% of the total body expression 5 and 14 days post injection, respectively), compared to Ad5CMV/Luciferase. treated mice (up to 1.8% of total body expression; Table 2). These results confirm the endothelial specificity observed in cell culture. It should be remembered that the liver is a highly vascularized organ. Therefore examination of cellular expression within organs was undertaken, as detailed hereinbelow.

**Table 2 - Luciferase expression in organs 5 and 14 days post injection of PPE-1 and CMV based constructs**

| Day post injection | 5 | | 14 | |
|---|---|---|---|---|
| | Light units/µg protein | | Light units/µg protein | |
| Organ | PPE-1 | CMV | PPE-1 | CMV |
| Aorta | 13.0±2.9 | 1.4±0.5 | 10.6±2.4 | 1.3±0.3 |
| | (32.7%) | (0.56%) | (12.6%) | (1.1%) |
| Heart | 02±0.1 | 1±0.6 | 1.5±0.3 | 1.8±0.6 |
| | (0.5%) | (0.4%) | (1.7%) | (1.6%) |
| liver | 22.7±4.5 | 219±111.5 | 34.9±7.8 | 52.8±10.6 |
| | (57%) | (88.6%) | (41.6%) | (46.8%) |
| lung | 0.2±0.1 | 2.3±1.0 | 3.6±0.8 | 2.0±0.9 |
| | (0.5%) | (0.9%) | (4.3%) | (1.8%) |
| muscle | 0.3±0.1 | 0.8±0.2 | 1.2±0.3 | 1.5±0.5 |
| | (0.7%) | (0.3%) | (1.4%) | (1.3%) |
| spleen | 1.3±0.8 | 1.6±0.9 | 2.0±0.4 | 2.3±0.9 |
| | (3.2%) | (0.6%) | (2.4%) | (2.0%) |
| pancreas | 2±0.6 | 20.1±6.8 | 26.4±5.9 | 452±24.5 |
| | (5.0%) | (8.1%) | (31.5%) | (40.1%) |
| kidney | 0.1±0 | 0.9±0.6 | 0.6±0.1 | 0.8±0.3 |
| | (0.25%) | (0.4%) | (0.71%) | (0.7%) |

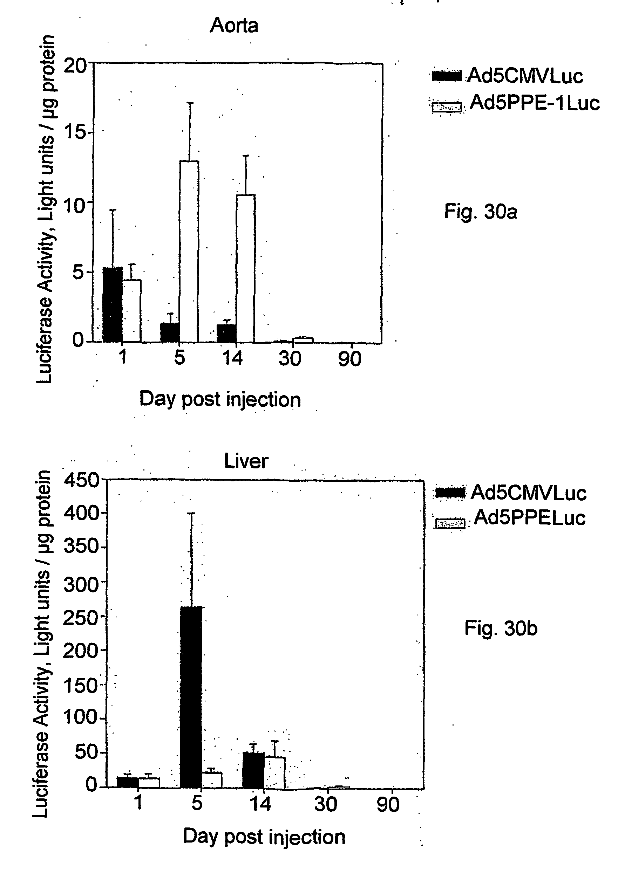

Figures 30A and 30B demonstrate the absolute Luciferase activity (light units/µg protein) in the aortas (A) and livers (B) of the 110 injected mice. Luciferase activity was measured 1 (n=13), 5 (n=34), 14 (n=32), 30 (n=20) and 90 (n=11)days post injection. The results in the aorta represent the promoters (PPE-1 or CMV) activity mostly in endothelial cells, while the results in the livers represent their activity mostly in hepatocytes.

### EXAMPLE 8

### Assays of efficiency, specificity and stability ofAd5PPE-1 in-vivo- in BALB/C mice

The experiments of example 7 were repeated in 12 week old BALB/C mice (n=10 for each group) in order to demonstrate that the observed results were not an artifact of a particular strain of animals.

Because Absolute results with the adenoviral vectors were lower in BALB/C mice than in C57BL/6 mice, the Luciferase expression is expressed as percentage of the total Luciferase activity in all tissues.

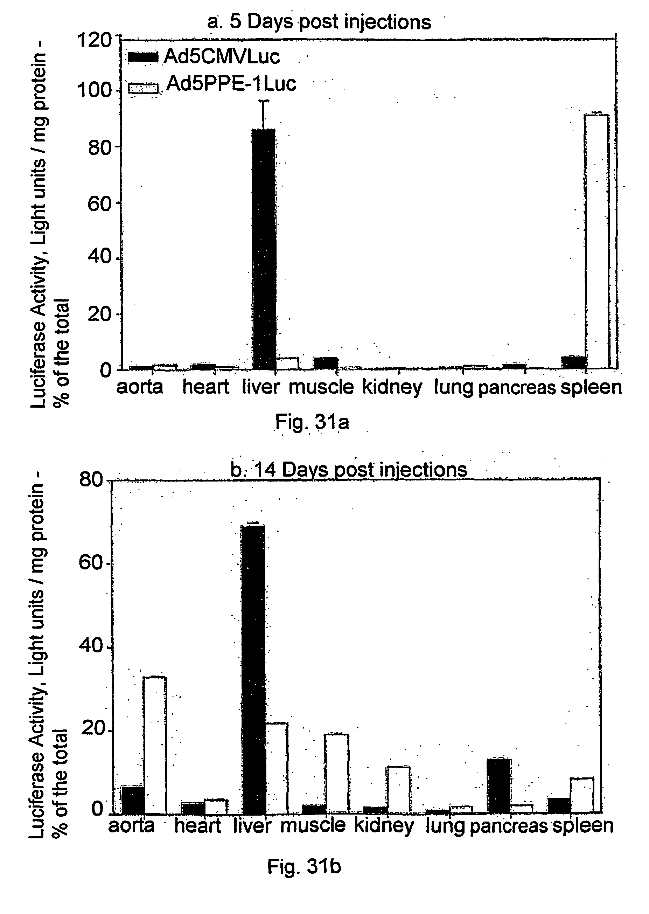

The highest relative Luciferase expression 5 days post injection was observed in the spleens of Ad5PPE-1 (90.9%), and in the livers of Ad5CMV (86.2%) injected mice. A significant increase in the relative Luciferase activity in the aortas of Ad5PPE-1 injected mice 14 days post injection (32.9%), compared to its activity five days post injection (1.75%) was also observed (Figures 31A and 31B; Ad5PPE-1Luc -open bars; Ad5CMVLuc-black bars).

These results confirm that regardless of mouse strain, the tissue specificity of the PPE-1 promoter is sufficiently strong to effectively eliminate hepatocyte expression, despite preferential uptake of injected DNA by hepatocytes.

### EXAMPLE 9

### Cellular localization of gene delivered by Ad5PPE-1 in-vivo

In order to ascertain cellular expression sites of the gene expressed by PPE-1 *in-vivo,* Green Fluorescent Protein (GFP) delivered by the adenoviral vector Ad5PPE-1-GFP was used. Ad5CMVGFP (Quantum, Canada) was used as non-endothelial-cell-specific negative control. Five days post-intravenous injection the mice were sacrificed and their tissues were analyzed by fluorescent microscopy.

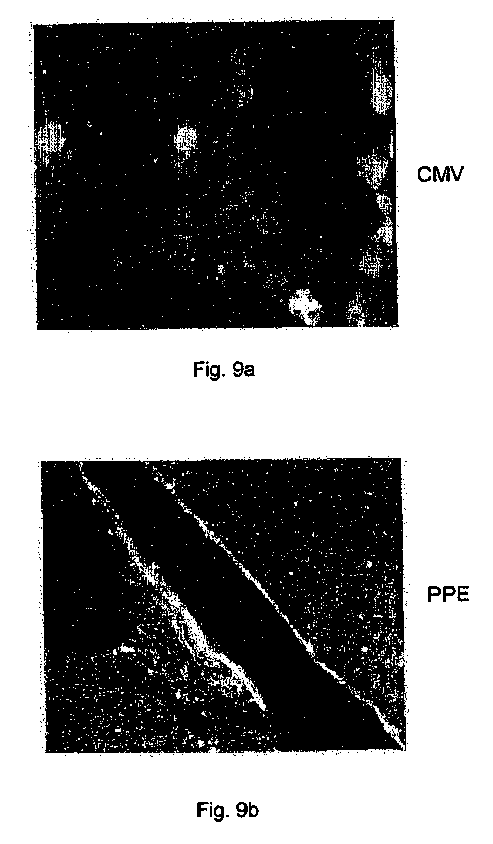

In the mice injected with Ad5CMVGFP vector, most of the expression was detected in the hepatocytes, and no expression was detected in endothelial cell in the liver (Figure 9A). In sharp contrast, Ad5PPE-1-GFP injected mice (Figure 9B), showed no expression in hepatocytes, but significant expression in endothelial cells in the blood vessels of the liver. Similar results were obtained in other tissues where practically all the PPE-1 derived expression was detected in the endothelium, while none of the CMV derived expression was endothelial. These results indicate endothelial specificity is preserved even within an organ containing endothelial and non-endothelial cells. This finding has important implications for prevention of angiogenesis in growing tumors.

### EXAMPLE 10

### Assays of efficiency and endothelial specificity of Ad5PPE-1-3X Luc and Ad5PPE-1-3X GFP in-vitro

In order to determine the relative efficacy of Ad5PPE-1 and Ad5PPE-1-3X in driving expression of the reporter genes Luciferase and green fluorescent protein (GFP) in cells, specific activity in endothelial cells was tested *in-vitro* using cell lines described hereinabove. Ad5CMVLuc and Ad5CMVGFP were employed as non-tissue specific controls. Ad5PPE-1Luc and Ad5PPE-1GFP were employed to ascertain the relative change in expression level caused by addition of the 3X sequence.

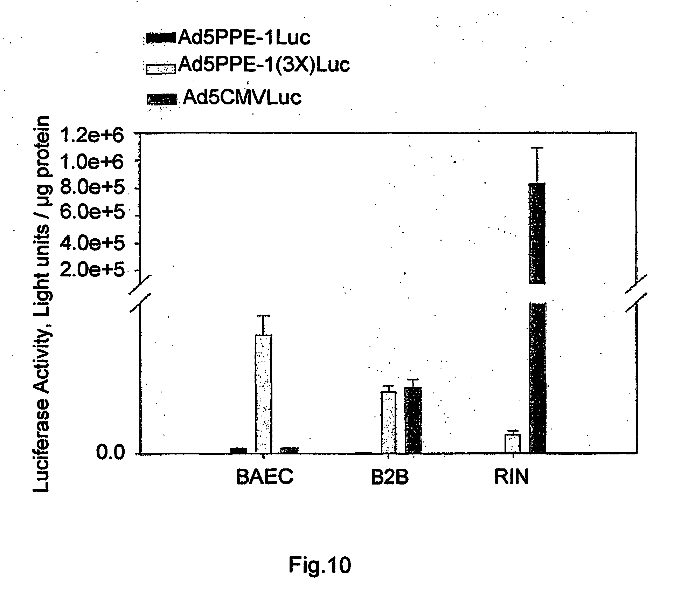

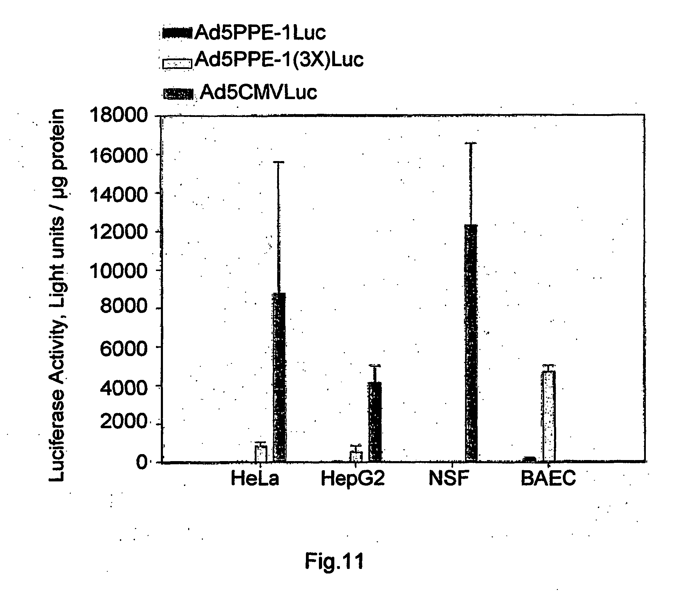

Results, summarized in Figures 10 and 11, indicate that Luciferase activities under the control of the PPE-1-3X promoter were 5-10 times higher in EC lines (Bovine Aortic Endothelial Cells - BAEC) compared to activity in non-endothelial cells - Rat Insulinoma - RIN, HeLA, HePG2 and normal skin fibroblasts (NSF) (figures 10 and 11).

Figure 10 shows Luciferase activity as light units/µg protein in B2B, BAEC and RIN cells transduced by Ad5PPE-1Luc, Ad5PPE-1-3XLuc, and Ad5CMVLuc Highest Luciferase expression was observed in RIN cells transduced by Ad5CMVLuc, however this construct was poorly expressed in BAEC and B2B cells. The next highest level of Luciferase expression was observed in BAEC cells transduced by Ad5PPE-1-3XLuc. Ad5PPE-1Luc was expressed at lower levels in BAEC cells. In the B2B cell line Ad5PPE-1Luc and Ad5PPE-1-3XLuc were expressed at nearly identical levels.

Overall, Luciferase activity in the endothelial cell lines under the control of PPE-1-3X promoter was 23 times higher than under the control of PPE-1 promoter and 23-47 times higher than under the control of the CMV promoter at the same infection conditions (moi=10). This is despite the fact that Luciferase expression in non-endothelial RIN cells was 3000 times higher under the control of the CMV promoter (figure 10).

In order to establish that PPE-1 and PPE-1-3X are inactive in other non-endothelial cell lineages HeLA, HepG2, NSF cell lines were transduced. BAEC was employed as an endothelial control. Figure 11 shows Luciferase activity as light units/µg protein in HeLA, HepG2, NSF and BAEC cells transduced by Ad5PPE-1Luc, Ad5PPE-1-3XLuc and Ad5CMVLuc. Transduction with Ad5CMVLuc caused high levels of Luciferase expression in HeLA, HepG2 and NSF cells. These cell lines failed to express Luciferase under the control of PPE-1 and expressed Luciferase at low levels with the PPE-1-3X promoter. As expected, BAEC cells transduced with Ad5PPE-1Luc or Ad5PPE-1-3XLuc exhibited high Luciferase expression.

Taken together these results indicate that introduction of the 3X sequence into the PPE-1 promoter caused higher levels of expression in endothelial cell lines while preventing unwanted expression in non-endothelial cells.

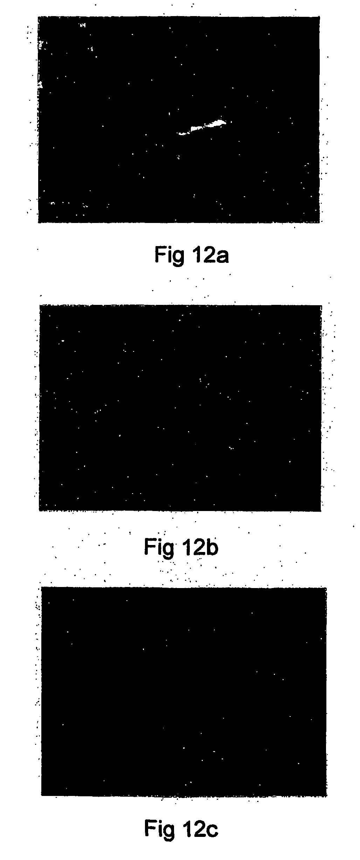

Addition of the 3X sequence to the PPE-1 promoter also increased levels of Green fluorescent protein expression in EC lines (Bovine Aortic Endothelial Cells - BAEC) as indicated in Figures 12A-C which depicts GFP expression in BAEC transduced by moi=1. No expression of GFP was observed using a CMV promoter in this experiment.

In Figure 12, panel A indicates Ad5PPE-1-3XGFP transduced cells, panel B indicates Ad5PPE-1GFP transduced cells and panel C indicates Ad5CMVGFP. Again, introduction of the 3X sequence into the PPE-1 promoter significantly increased expression of the reporter gene. This result indicates that the ability of the 3X sequence to function as an endothelial specific enhancer is not a function of the downstream gene being transcribed.

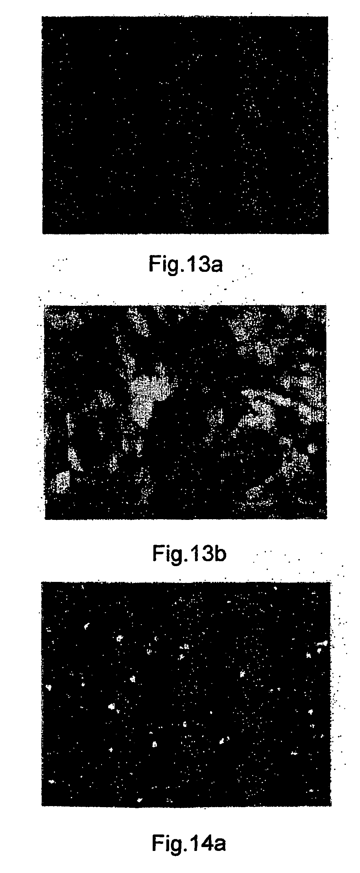

Moreover, Ad5PPE-1-3X-GFP and Ad5PPE-1GFP transduction resulted in no GFP expression in non-endothelial cells SMC, HelA, HePG2 and normal skin fibroblasts (NSF) compared to the high expression under the CMV promoter as summarized in Figures 13-16.

Figure 13 shows GFP expression in SMC transduced by moi=1 of either Ad5PPE-1-3XGFP (panel A) or Ad5CMVGFP (panel B). While high level GFP expression resulted from Ad5CMVGFP transduction, no GFP expression resulted from transduction with Ad5PPE-1-3XGFP transduction.

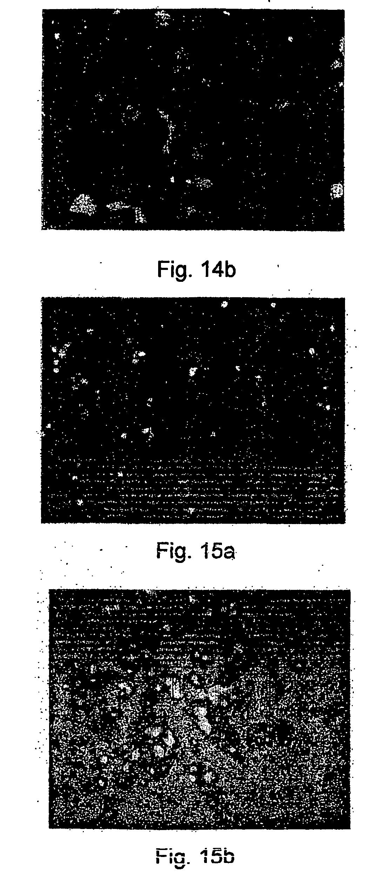

Figure 14 shows results of a similar experiment conducted in HeLa cells. As in the previous figure, panel A indicates cells transduced with Ad5PPE-1-3XGFP and panel B indicates cells transduced with Ad5CMVGFP. Again, while high level GFP expression resulted from Ad5CMVGFP transduction, no GFP expression resulted from transduction with Ad5PPE-1-3XGFP transduction.

Figure 15 shows results of a similar experiment conducted in HepG2 cells. As in the previous figure, panel A indicates cells transduced with Ad5PPE-1(3X)GFP and panel B indicates cells transduced with Ad5CMVGFP. Again, while high level GFP expression resulted from Ad5CMVGFP transduction, no GFP expression resulted from transduction with Ad5PPE-1-3XGFP.

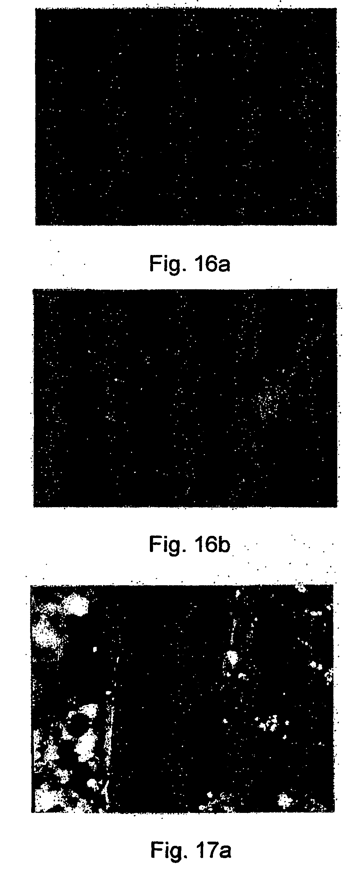

Figure 16 shows results of a similar experiment conducted in NSF cells. As in the previous figure, panel A indicates cells transduced with Ad5PPE-1-3XGFP and panel B indicates cells transduced with Ad5CMVGFP. Again, while high level GFP expression resulted from Ad5CMVGFP transduction, very low GFP expression resulted from transduction with Ad5PPE-1-3XGFP.

These results, taken together, indicate a high level of endothelial specificity and a high level of endothelial expression is obtained by using a modified PPE-1 promoter containing the 3X sequence of SEQ ID NO.: 7.

### EXAMPLE 11

### Cellular localization of a reporter gene delivered by Ad5PPE-1-3X in-vivo

In order to determine the cellular localization pattern of a reporter gene expressed under the control of the PPE-1-3X promoter *in-vivo*, Ad5PPE-1-3XGFP and Ad5PPE-1GFP were injected into mice as described hereinabove. Five days post-intravenous injection, the mice were sacrificed and their tissues were analyzed by a fluorescent microscopy.

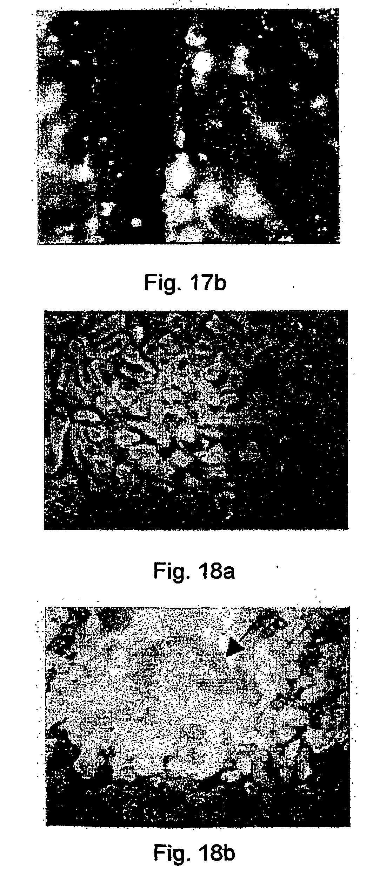

Significantly higher GFP activity was observed in the endothelial cells of the liver, kidney and spleen blood vessels of Ad5PPE-1-3XGFP injected mice compared to the Ad5PPE-1GFP injected mice. Figures 17A and B show representative results.

Figure 17A shows low level GFP expression in endothelial cells lining a blood vessel of a mouse injected with the Ad5PPE-1GFP. Figure 17B shows the much higher level of GFP expression resulting from addition of the 3X sequence to the construct.

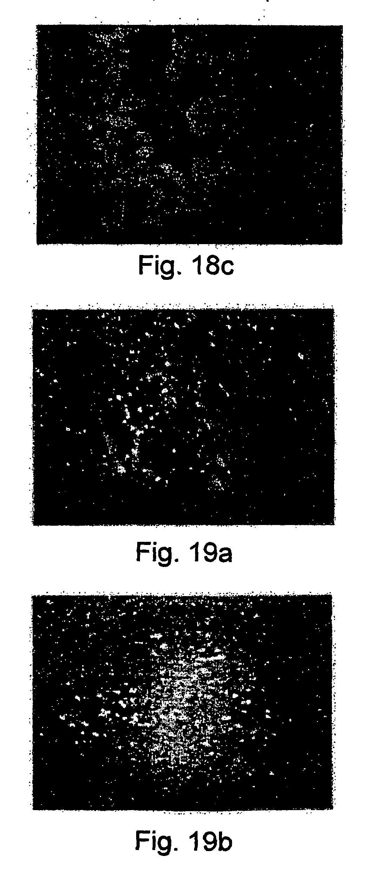

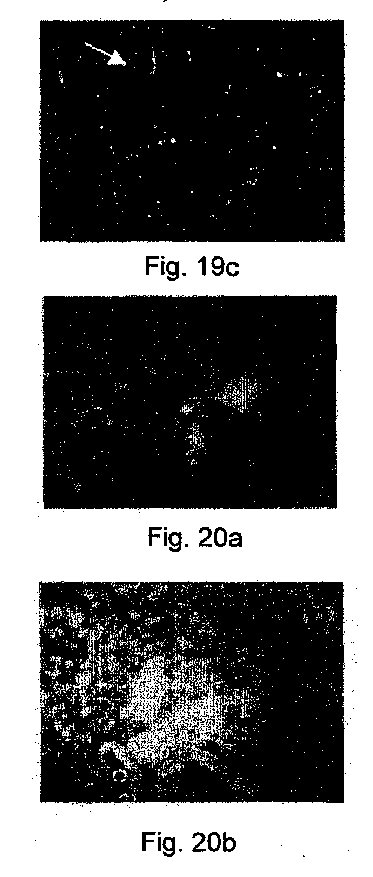

Despite the high expression in the lining of the blood vessels, no expression was detected in the hepatocytes, glomeruli, epithelial cells and splenocytes (Figures 18 and 19).

Figure 18 shows representative results from kidney tissue of injected mice. Ad5CMVGFP injected mice (Figure 18A), Ad5PPE-1GFP (Figure 18b) and Ad5PPE-1-3XGFP (Figure 18C) injected mice all exhibited low GFP activity in kidney cells. In figure 18B, slightly higher GFP expression is visible in the blood vessel wall (indicated by arrow).

Figure 19 shows representative results from spleen tissue of injected mice. Ad5CWGFP injected mice(Figure 19A), Ad5PPE-1GFP injected mice (Figure 19B) and Ad5PPE-1-3XGFP injected mice (Figure 19 C) all exhibited low level GFP activity in cells of the spleen. Higher GFP activity is visible in the blood vessels of Ad5PPE-1-3XGFP injected mice (indicated by arrow).

These results confirmed that both the PPE-1 and the PPE-1-3X promoter are endothelial cell specific *in-vivo.* They further suggest that activity of both promoters was limited in non-proliferating endothelial tissue (i.e. blood vessels of healthy organs. Therefore, assays in a tumor angiogenic model were undertaken.

### EXAMPLE 12

### Assays of the Ad5PPE-1 construct in tumor neovascularization in-vivo

In order to ascertain the ability of AD5PPE to specifically direct expression of a reporter gene to angiogenic blood vessels in a tumor, the murine LLC model (described hereinabove in materials and methods) was employed.

In a one experiment, Luciferase expression in tumor neovascularization was tested five days post systemic injections of Ad5PPE-1Luc or Ad5CMVLuc (10¹⁰pfu/ml each).

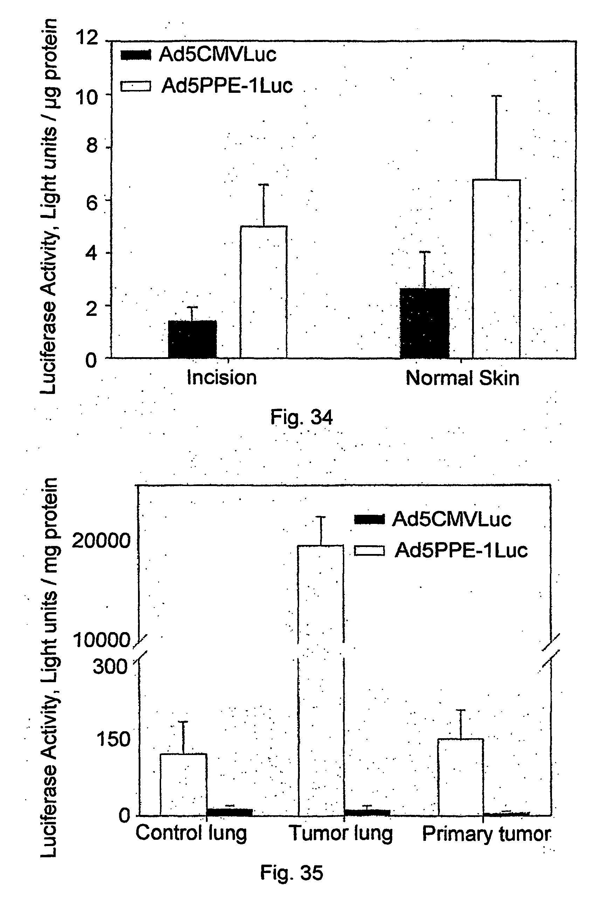

In this experiment, systemic injection of Ad5CMVLuc to both primary and metastatic tumor models resulted in minimal expression in the primary tumor or in the metastatic lung. This level of expression was similar to the minimal expression of Luciferase directed by CMV in naive normal lungs (Figure 35; black bars; n=12). In sharp contrast, under the control of PPE-1 promoter (Figure 35; open bars; n=9), the highly angiogenic lung metastases were associated Luciferase activity which was about 200 times higher than the Luciferase activity in the poorly-vascularized primary tumor and the naive lungs.

The Luciferase expression in non-metastatic tissues such as the liver, kidney, heart and pancreas was minimal. The expression level in the aorta was about 30% of the levels in the metastatic lungs.

In an additional experiment in the LLC model Ad5PPE-1GFP and Ad5CMVGFP constructs were employed to localize reporter gene expression in the primary tumor and metastatic lungs.

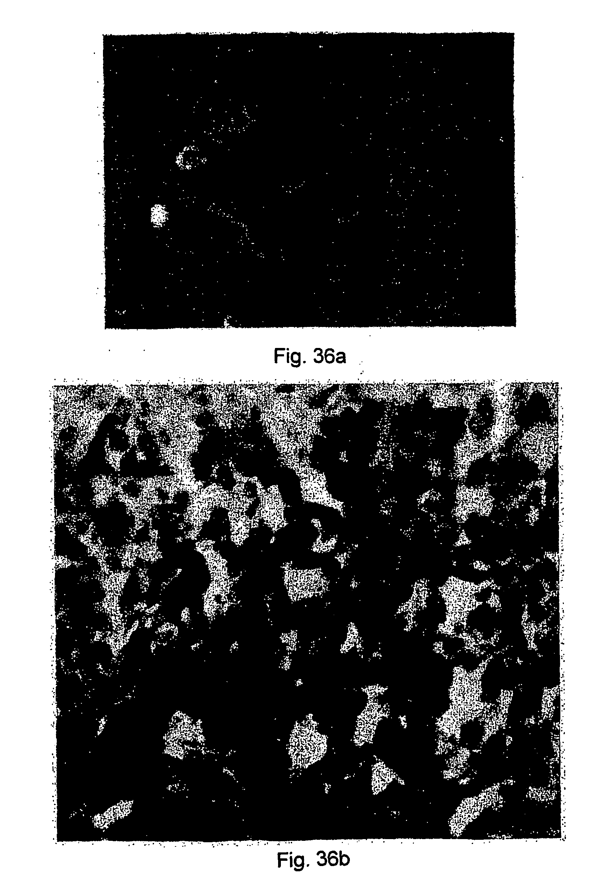

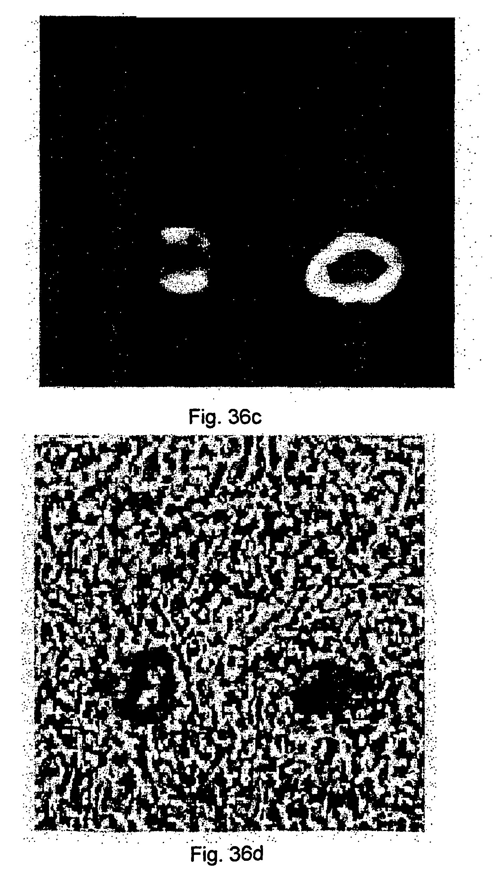

Ad5PPE-1GFP injected mice, showed high levels of GFP specific expression in the blood vessels of the primary tumor (Figure 36C), although no expression was detected in the tumor cells themselves. This observation is consistent with the results of the LLC cell culture model presented in example 20. In lung metastases, high levels of GFP expression were detected in both big arteries and small angiogenic vessels of the metastatic foci (Figure 36A). No expression was detected in the normal lung tissue. The endothelial cell localization was demonstrated by co-localization of the GFP expression (Figure 16A) and the CD31 antibody immuno-staining (Figure 16B). In striking contrast, in Ad5CMVGFP injected mice, no GFP activity was detectable in both the primary tumor and lung metastasis.

Figure 36C illustrates GFP expression in blood vessels of a primary tumor after intra tumoral injection of Ad5PPE-1GFP. Figure 36D is a phase contrast image of the same filed as panel C illustrating the tumor and its blood vessels.

These results indicate that while PPE-1 does not drive high level expression in tumor cells per se, the promoter does drive high level expression in vascular endothelia within the tumor, especially in rapidly proliferating angiogenic vessels.

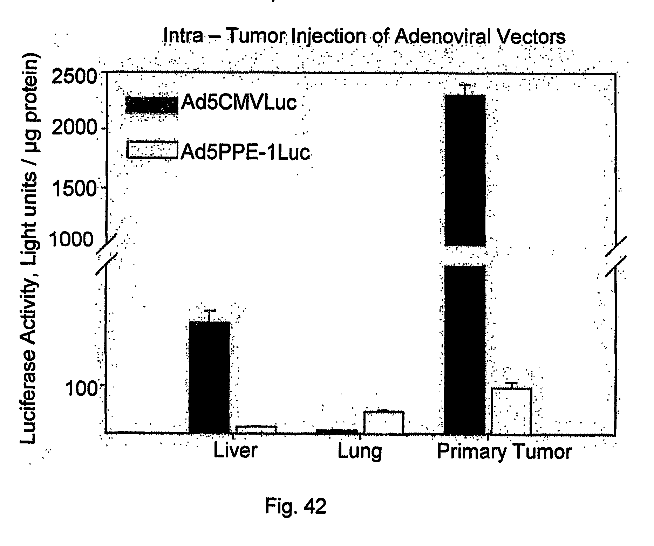

Intra-tumor injection of Ad5CMV into primary subcutaneous tumor model resulted in high Luciferase expression in the tumor tissue and moderately levels of expression liver (10% of the amount expressed in the tumor; Figure 42). No expression was detected in the metastatic lungs. On the other hand, when injected intra-tumoral, Luciferase expression under the control PPE-1 promoter resulted in similar Luciferase levels of expression in the primary tumor and the metastatic lungs and no expression was detected in the liver.

### EXAMPLE 13

### Assays of the Ad5PPE-1 construct in a carcinoma cell culture system

In order to assay the efficiency of Ad5PPE-1 and Ad5CMV to drive Luciferase expression in cancerous cells, the D122-96 Lewis Lung Carcinoma cell line was employed.

*In-vitro* transduction at varying multiplicities of infection (moi) was performed. The results indicate that both adenoviral vectors are able to transduce the Luciferase gene to these cells (Table 3). Nevertheless, Luciferase activity directed by the PPE-1 promoter was much lower in the LLC cells than the activity detected in endothelial cells, 50 vs. 1000-2500 light units/µg protein, respectively.

**Table 3 - In-vitro transduction of Lewis lung carcinoma cell line (D122-96) with Ad5PPE-1Luc and Ad5CMVLuc.**

| | **MOI=1** | **MOI=5** | **MOI=10** |
|---|---|---|---|
| ***Ad5PPE-1*** | 8.1±0.06 | 33.95±7.0 | 50.7±5.0 |
| **Ad5CMV** | 9.3±1.1 | 47.3±4.0 | 88.13±10.1 |

### EXAMPLE 14

### Assay of the effect of the 3X sequence in tumor angiogenic blood vessels in-vivo

In order to ascertain the effect of the 3X sequence on the PPE-1 promoter in angiogenic blood vessels, the Lewis Lung Carcinoma (LLC) metastases model (described hereinabove in material and methods) was employed. Five days post IV injection of 10¹⁰ infectious units of Ad5PPE-1GFP, Ad5PPE-1-3XGFP or Ad5CMVGFP, the mice were sacrificed and their tissues were analyzed as described in material and methods.

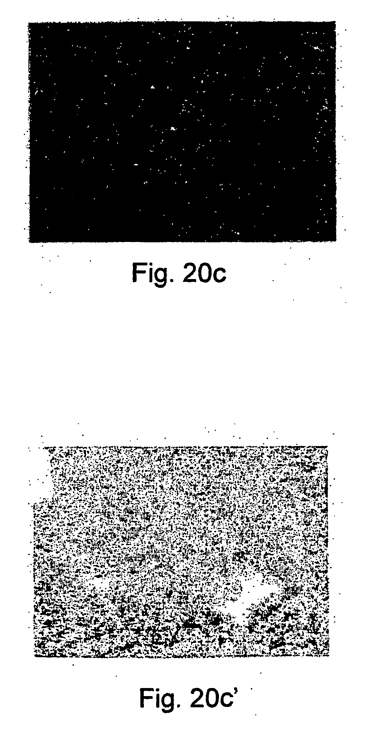

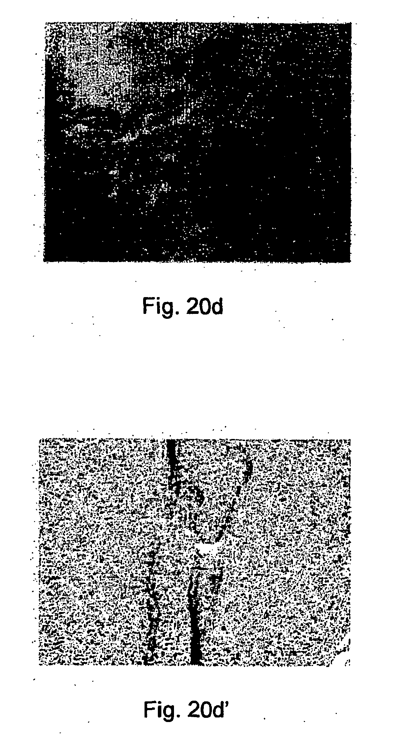

Figures 20A-D summarize the GFP expression in metastatic lungs of control mice injected with Saline (Figure 20A), mice injected with Ad5CMVGFP (Figure 20 B), mice injected with Ad5PPE-1GFP (Figure 20 C) and mice injected with Ad5PPE-1-3XGFP (Figure 20D). Anti-CD31 immunostaining (Figures 20C' to 20D') confirm the location of the GFP expression in each metastatic tissue. The results show that while no GFP expression was detected in control - saline injected mice (Figure 20A), there was a slight expression around the epithelial bronchi of the CMV injected mice, but not in the angiogenic blood vessels of the metastatic lung of these mice (Figure 20B). Low GFP expression was observed in metastatic lungs of Ad5PPE-1GFP injected mice (Figures 20C and 20C'), while high and specific expression was observed in the new blood vessels of Ad5PPE-1-3XGFP injected mice (Figure 20D and 20 D').

These results explain the apparent disparity between the in-vivo results of example 10 and the *in-vitro* results of examples 2, 3 and 6. Both the PPE-1 and the PPE-1-3X promoter are endothelial specific. However, the 3X sequence greatly increases the level of expression in rapidly proliferating endothelial tissue, such as newly forming blood vessels in a growing tumor.

### EXAMPLE 15

### Effect of the 3X element on the PPE-1 promoter in tumor angiogenic blood vessels

In order to study the effect of the 3X element of the present invention on efficacy and specific activity of the PPE-1 promoter in tumor angiogenic blood vessels, the LLC metastases model was employed. Five days post i.v. injection of 10¹⁰ pfu/ml of Ad5PPE-1Luc, Ad5PPE-1-3XLuc, Ad5CMVLuc, Ad5PPE-1GFP, Ad5PPE-1-3X-GFP or Ad5CMVGFP, the mice were sacrificed and their tissues were analyzed for Luciferase or GFP expression as described hereinabove.

Figure 37 is a histogram comparing Luciferase expression in normal lungs versus that in metastatic lungs after systemic injection of Ad5PPE-1-3X1uc, Ad5PPE-1Luc or Ad5CMVLuc. Experimental groups were Ad5CMVLuc (n=7; black bars), Ad5PPE-1Luc (n=6;gray bars) and Ad5PPE-1-3XLuc (n=13; brown bars). Activity is expressed as light units/µg protein.

Luciferase expression under the control of the PPE-1-3X promoter was 35 fold greater in the metastatic lungs relative to its activity in normal lungs and 3.5 fold higher than expression driven by the PPE-1 promoter without the 3X element (p<0.001). Very low Luciferase activity was detected in other tissues of mice injected with Ad5PPE-1-3XLuc. Calculating the Luciferase expression in the lungs as percentage from the liver of each injected animal revealed that the activity increased 10 fold in the metastatic lung compared to the activity in normal lung (Figure 38).

In order to localize reporter gene expression to specific cell types, GFP constructs were employed. The GFP expression in metastatic lungs of Ad5PPE-1-3XGFP injected mice has been shown. Immuno-staining by CD31 antibody conform the location of the GFP expression in the new blood vessels. No GFP expression was detected in control - saline injected mice. Low level expression around the epithelial bronchi of the CMV injected mice, but not in the angiogenic blood vessels of the metastatic lung. In summary, these results indicate that large increases in expression level resulted from introduction of a 3X element into Ad5PPE-1 constructs and that this increased expression was specific to the angiogenic blood vessels of tumors. Potentially, the observed effect may be coupled with the hypoxia response described hereinabove to further boost expression levels of a sequence of interest.

### EXAMPLE 16

### Further characterization of the PPE-1 hypoxia response

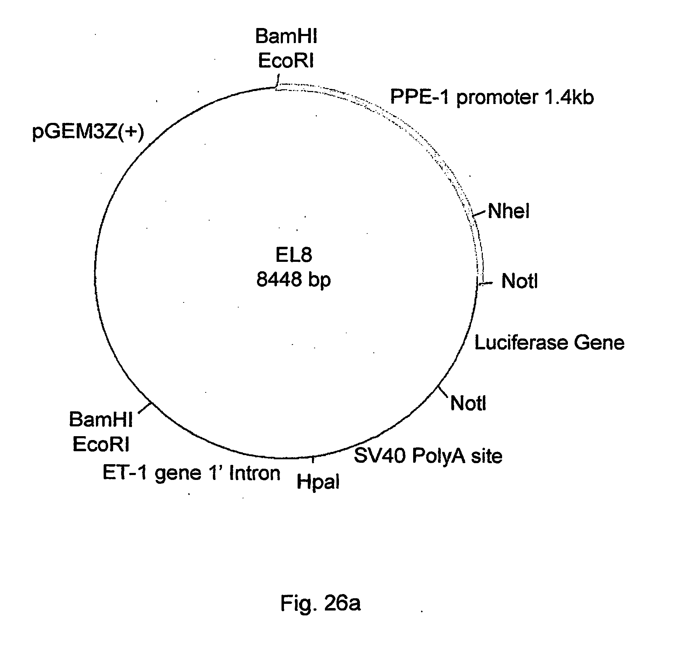

In order to further characterize the effect of hypoxia on the murine PPE-1 promoter activity, bovine aortic endothelial cells (BAEC) were transfected by a DNA plasmid (pEL8; Figure 26A). The pEL8 plasmid contains the murine PPE-1 promoter (1.4kb) (red), the luciferase gene (1842 bp), the SV40 poly A sites and the first intron of the endothelin-1 gene, all termed the PPE-1 promoter cassette was digested and extracted by BamHI restriction enzyme as described in material and methods. After transfection, cells were subjected to hypoxic conditions.

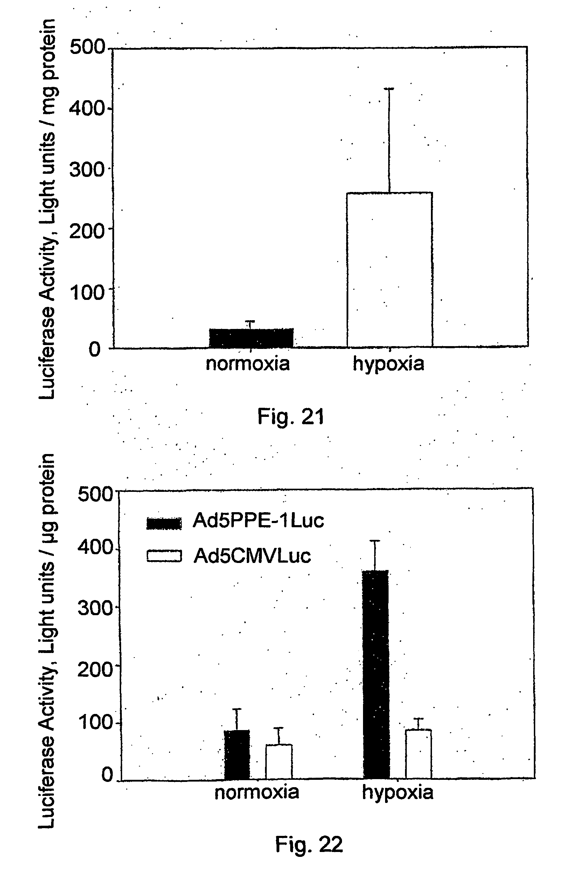

Luciferase expression in transfected BAEC subjected to 18 hours of hypoxia (0.5% 02) was eight times higher than Luciferase expression in cells grown in a normoxic environment (Figure 21). Figure 21 shows that Luciferase activity (light units/µg protein) in BAEC transfected by a plasmid containing the murine PPE-1 promoter was significantly higher when transfected cells were incubated in a hypoxic environment. Equivalent transfection efficiencies were confirmed by co-transfection with a β-galactosidase reporter vector and assays of LacZ activity.

In order to determine whether murine PPE-1 promoter delivered by adenoviral vector is also up-regulated by hypoxia, BAEC were transduced by Ad5PPE-1Luc. Ad5CMVLuc was used a non specific control in this experiment. Results are summarized in Figure 22. Hypoxia Luciferase activity in BAEC transduced by Ad5PPE-1Luc. In stark contrast, no significant difference between normoxia and hypoxia was detected in the Ad5CMV transduced cells (Figure 22).

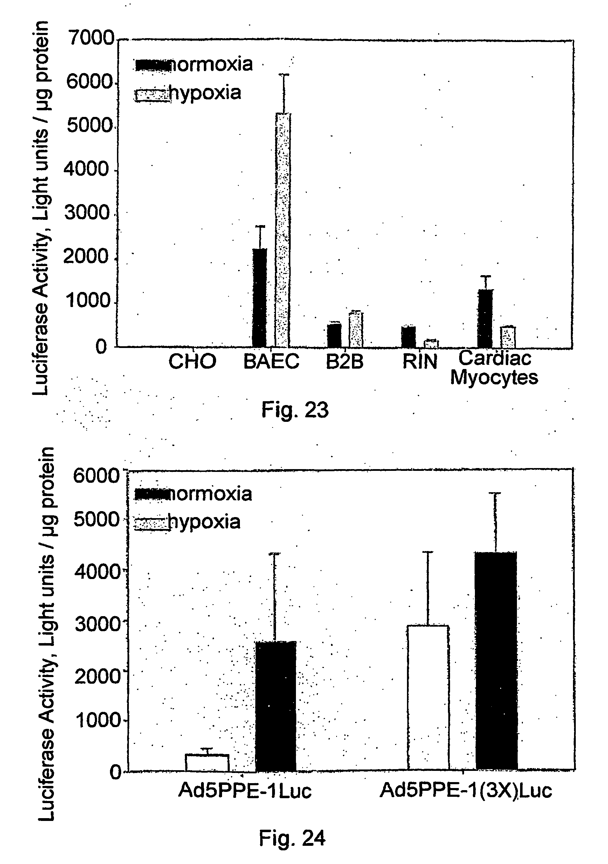

To understand whether the enhancement of the PPE-1 promoter activity is specific to endothelial cells, different cell lines (BAEC, B2B, CHO, RIN and Cardiac Myocytes) were transduced by Ad5PPE-1 (moi=10) and were subjected to hypoxia (0.5% 02) or normoxia environment. Results are summarized in figure 23. Luciferase expression was slightly increased in B2B cells and significantly increased in BAEC cells cultured in a hypoxic environment. Luciferase expression in other cell lines was reduced by the hypoxic environment, compared to normoxia. These results confirm that hypoxic induction of the PPE-1 promoter occurs primarily in endothelial cell lineages.

### EXAMPLE 17

### Effect of the 3X sequence on the PPE-1 hypoxia response

In order to ascertain the effect of the 3X sequence on the PPE-1 hypoxia response, BAEC were transduced by Ad5PPE-1Luc and Ad5PPE-1(3X)Luc. Following transduction, the BAEC cells were incubated either in a hypoxic or a normoxic environment as detailed hereinabove. Results are summarized in Figure 24. Luciferase expression using the Ad5PPE-1Luc construct significantly increased (seven folds) in response to hypoxia (2578 in hypoxia and 322.1 in normoxia). In contrast, the Ad5PPE-1(3X)Luc construct exhibited only 1.5 fold increase in response to hypoxia (from 2874.5 in normoxia to 4315 in hypoxia conditions). These results indicate that the high normoxic level of expression observed when the 3X sequence is added to the PPE-1 promoter serves to mask the hypoxic response to some extent.

### EXAMPLE 18

### Assays of the PPE-1 response to hypoxia in a transgenic mouse model

In order to examine the murine PPE-1 promoter activity in tissues subjected to regional hypoxia/ischemia, mPPE-1-Luc transgenic mice, described hereinabove in materials and methods, were employed. The mice were induced to regional hind limb ischemia as previously described (Couffinhal T. et al. (1998) Am. J. Pathol. 152;1667-1679). In brief, animals were anesthetized with pentobarbital sodium (40 mg/kg, IP). Unilateral ischemia of the hind limb was induced by ligation of the right femoral artery, approx. 2 mm proximal to the bifurcation of the saphenous and popliteal arteries. To verify the induction of functional change in perfusion, ultrasonic imaging was performed on days 4 and 14 by Synergy ultrasound system (GE) equipped with a 7.5 MHz transducer and angiographic software. Animals were housed under conventional conditions for up to 18 days.

Luciferase expression was assayed 2, 5, 10 and 18 days post ligation in the ischemic muscle, in the normal non-ligated muscle, in the liver, lung, and aorta.

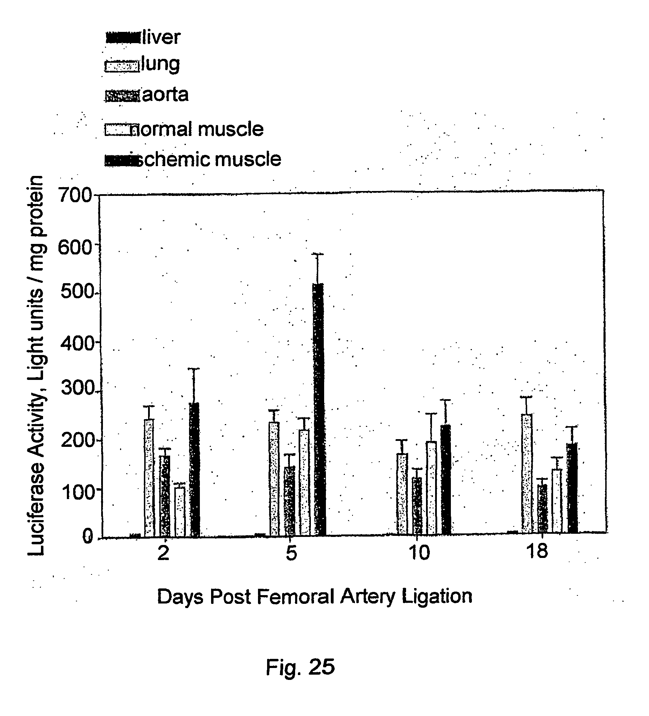

Results, summarized in Figure 25, show that while no significant difference was detected in the liver, lung and aorta during the days post ligation, Luciferase gene expression increased after the femoral ligation in both in the normal non-ligated and in the ischemic muscle. While peak Luciferase expression in the ischemic muscle was detected five days post ligation, peak Luciferase expression in the non-ligated muscle was detected ten days post femoral artery ligation. This indicates that the hypoxic response of the PPE-1 promoter is functional in an in-vivo system. Luciferase expression in the non-ischemic muscle did not change during the days tested, compared to its expression in the control non-operated tissue (day=0). In contrast, Luciferase expression in the ischemic muscle was significantly higher on day 5 than at other time points.

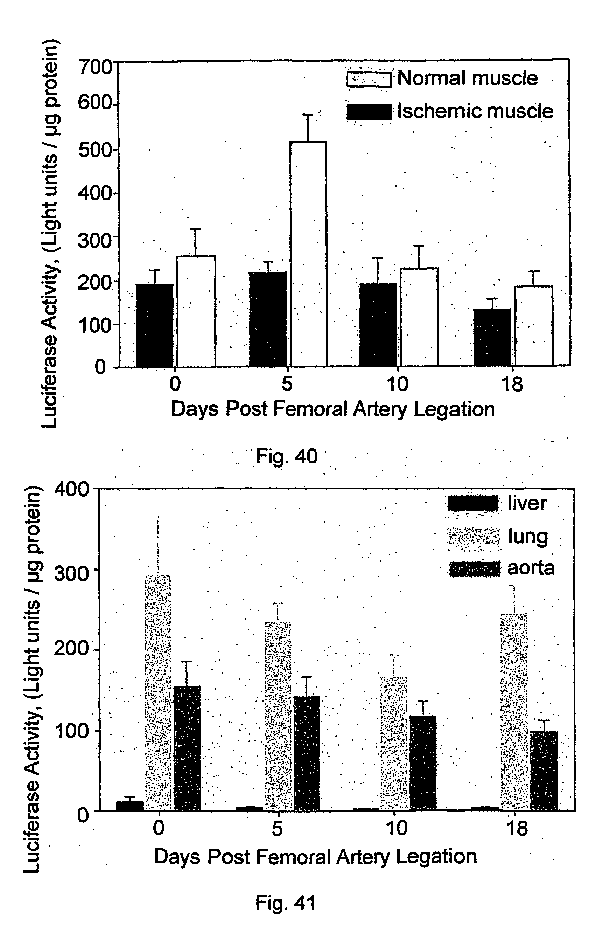

On day 5, PPE-1 driven expression of Luciferase was 2.5 times higher than in control non-operated mice and compared to the ischemic muscle in days 10 and 18 (Figure 40).

Expression of Luciferase in other non-ischemic tissues including liver, lungs and aorta of the transgenic mice subjected to regional ischemia revealed no significant changes within 18 days post ischemic induction in the Luciferase expression in these tissues (Figure 41).

Further, these results confirm that Luciferase expression was higher in tissues containing a high percentage of endothelial tissue (lung and aorta) than in those tissues containing a low percentage of endothelial tissue (liver and non-ischemic muscle).

### EXAMPLE 19

### Effect of level of cellular proliferation on Ad5PPE-1Luc activity in endothelial cells

In order to ascertain the effect of level of cellular proliferation on efficiency and specific activity of Ad5PPE-1Luc, an angiogenic model of endothelial cells (BAEC), was tested *in-vitro*. Transduced BAEC were either induced to quiescence by serum deprivation or grown in 10% FCS for normal proliferation. Briefly, cells were transduced for 48 hours either as quiescent cells - 72 hours post serum deprivation or as proliferating cells - in normal media (10% FCS). Luciferase activity is expressed as light unit/µg protein, to normalize for the difference in cell amount. The results presented are an average of triplicate test from four representative independent experiments.

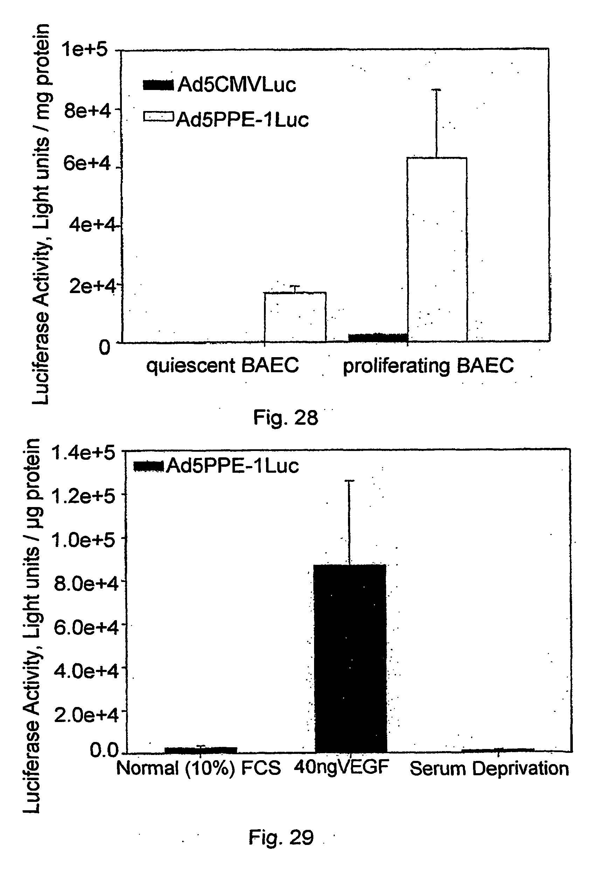

Luciferase expression under the control of PPE-1 promoter (open bars; Figure 28) was 4 times higher in normal proliferating BAEC than in quiescent cells, and 25 times higher in normal proliferating BAEC than Luciferase expression under control of the CMV promoter (Black bars; Figure 28). Further, in proliferating cells, the activity under the control of PPE-1 promoter was 10 times higher than that under the CMV promoter control.

In order to simulate angiogenic conditions *in-vitro*, Ad5PPE-1Luc activity was tested in BAEC induced to rapid proliferation by addition of 40 ng/ml vascular endothelial growth factor (VEGF). Activity under these conditions was compared activity in normal proliferating cells and quiescent cells as described hereinabove. Luciferase expression in BAEC induced to cell proliferation with VEGF was 44 times higher than in normal proliferating cells, and 83 times higher than in quiescent cells (Figure 29).

Together, these experiments indicate that the level of activity of a sequence of interest under transcriptional control of the PPE-1 Promoter is a function of the level of cellular proliferation, with rapid proliferation causing higher levels of expression.

### EXAMPLE 20

### Assays of the PPE-1 promoter in Atherosclerosis induced mice

In order to test the efficiency and specificity of the Ad5PPE-1 vector in atherosclerotic blood vessels, 10¹⁰pfu/ml of the viral vectors were systemically injected to 6 month old ApoE deficient mice (Plump, A.S. et al. Cell; 1991; 71:343-353).

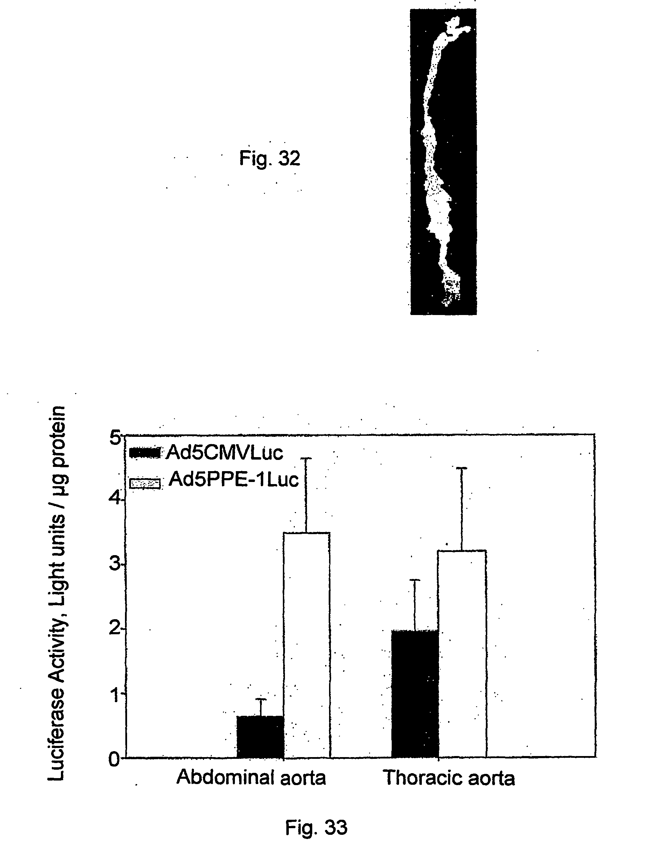

As ApoE deficient mice age, they develop high cholesterol values and extensive atherogenic plaques with no induction of lipid reach diet. Figure 32 is a picture of an aorta dissected from an ApoE deficient mouse colored by Sudan - IV. Note that the thoracic aorta contains less red stained atherosclerotic lesions while the abdominal region is highly atherosclerotic. (Figure 32 adapted from Imaging of Aortic atherosclerotic lesions by 125I-HDL and 125I-BSA. A. Shaish et al, Pathobiology - submitted for publication).

Figure 33 summarizes Luciferase expression observed 5 days post systemic injections of Ad5PPE-1Luc (open bars; n=12) and Ad5CMVLuc (black bars; n=12) to ApoE deficient mice. Results are presented as absolute Luciferase expression in the thoracic area that contains less atherosclerotic lesion, and the abdominal aorta that is rich atherosclerotic lesion.

Luciferase expression controlled by the PPE-1 promoter was 6 fold higher in the highly atherosclerotic abdominal, and 1.6 fold higher in the slightly atherosclerotic thoracic aorta as compared to expression under the control CMV promoter.

No significant difference was observed between the two aorta regions in the Ad5PPE-1Luc injected mice, while higher Luciferase expression was observed in thoracic aorta of the Ad5CMVLuc injected group compared to low expression in the abdominal aorta that contain lesion.

These results indicate that while a constitutive promoter (CMV) has a tendency to shut down in areas where atherosclerosis is most severe, the PPE-1 promoter is relatively unaffected by disease progression.

### EXAMPLE 21

### Assays of the PPE-1 promoter in a wound healing model

In order to test the Ad5PPE-1 constructs efficiency and specific activity in directing Luciferase expression to healing wound blood vessels, a murine wound healing as described hereinabove in Material and Methods was employed.

As in other experiments, Ad5CMVLuc was used as a non-tissue specific control. Luciferase activity under the PPE-1 promoter (Figure 34; open bars) control was higher both in the normal (6.8±3.2) and in healing wound region (5±1.6) compared to the activity observed under the CMV control (Figure 34; black bars).

Because both the CMV and PPE-1 promoter exhibited reduced expression levels in the healing wound, these results are difficult to interpret. Despite this unexpected observation, it is clear that the PPE-1 promoter drives higher levels of expression than the CMV promoter in both normal and healing tissue. The presence of necrotic scar tissue may account for the reduced expression levels observed with both promoters in the healing wound.

## Claims

1. An isolated polynucleotide functional as a regulatory element, the isolated polynucleotide comprising at least one copy of the sequence set forth in SEQ ID NO:8, said regulatory element being capable of regulating expression of a nucleotide sequence operably linked to a promoter functional in endothelial cells.

2. The isolated polynucleotide of claim 1, wherein said regulatory element further includes at least one copy of the sequence set forth in SEQ ID NO:6.

3. The isolated polynucleotide of claim 1, wherein said regulatory element includes one copy of the sequence set forth in SEQ ID NO:8 and at least two copies of the sequence set forth in SEQ ID NO:6.

4. The isolated polynucleotide of claim 1, wherein said promoter functional in endothelial cells comprises at least one copy of the PPE-1 promoter.

5. The isolated polynucleotide of claim 1, further comprising a hypoxia response element.

6. The isolated polynucleotide of claim 5, wherein said hypoxia response element includes at least one copy of the sequence set forth in SEQ ID NO: 5.

7. The isolated polynucleotide of claim 1, wherein said regulatory element is as set forth in SEQ ID NO: 7.

8. A nucleic acid construct comprising the isolated polynucleotide of claim 1 and a nucleic acid sequence of interest, said nucleic acid sequence of interest being under regulatory control of said isolated polynucleotide.

9. The nucleic acid construct of claim 8, wherein said nucleic acid sequence of interest is selected from the group consisting of VEGF, p55 and PDGF-BB.

10. A mammalian cell transformed with the isolated polynucleotide of claim 1.

11. Use of a construct functional in endothelial cells for the manufacture of a medicament for the expression of a nucleic acid sequence of interest in endothelial cells, said construct comprising a nucleic acid sequence of interest positioned under the regulatory control of a promoter functional in endothelial cells, and a regulatory element including at least one copy of the sequence set forth in SEQ ID NO:8.

12. The use of 11, wherein said promoter exhibits endothelial cell specificity.

13. The use claim 12, wherein said promoter is the PPE-1 promoter as set forth in SEQ ID NO: 1.

14. The use claim 11, wherein said regulatory element further includes at least one copy of the sequence set forth in SEQ ID NO:6.

15. The use claim 14, wherein said regulatory element is as set forth in SEQ ID NO: 7.

16. The use claim 14, wherein said at least one copy of the sequence set forth in SEQ ID NO:6 includes two copies.

17. The use claim 14, wherein said at least two copies of the sequence set forth in SEQ ID NO:6 are contiguous.

18. The use claim 11, wherein said nucleic acid sequence of interest is selected from the group consisting of VEGF, p55 and PDGF-BB.

19. The use claim 11, wherein said medicament is designed for administration by a method selected from the group consisting of:
(i) systemic in-vivo administration;
(ii) ex-vivo administration to cells removed from a body of a subject and subsequent reintroduction of said cells into said body of said subject; and
(iii) local in-vivo administration.

20. Use of a nucleic acid construct for the manufacture of a medicament for regulating angiogenesis in a tissue, the nucleic acid construct comprising:
(a) an endothelial cell specific promoter;
(b) at least one copy of a hypoxia response element set forth in SEQ ID NO:5;
(c) a regulatory element including at least one copy of the sequence set forth in SEQ ID NO:8; and
(d) a nucleic acid sequence encoding an angiogenesis regulator, said nucleic acid sequence being under regulatory control of said promoter and said hypoxia response element.

21. The use of claim 20, wherein said medicament is designed for administration by a method selected from the group consisting of:
(i) systemic in-vivo administration;
(ii) ex-vivo administration to cells removed from a body of a subject, said cells subsequently reintroduced into said body of said subject; and
(iii) local in-vivo administration.

22. The use of claim 20, wherein said nucleic acid construct further includes;
(e) at least one copy of a sequence set forth in SEQ ID NO: 6.

23. The use of claim 22, wherein said at least one copy of a sequence set forth in SEQ ID NO: 6 includes two contiguous copies.

24. The use of claim 20, wherein said endothelial cell specific promoter comprises at least one copy of the PPE-1 promoter.

25. The use of claim 20, wherein said nucleic acid sequence encoding an angiogenesis regulator is selected from the group consisting of VEGF, p55 and PDGF-BB.

26. An isolated polynucleotide functional as a promoter in eukaryotic cells, the isolated polynucleotide comprising a regulatory element including the sequence set forth in SEQ ID NO: 7.

27. The isolated polynucleotide of claim 26, further comprising an endothelial specific promoter element.

28. The isolated polynucleotide of claim 27, wherein said endothelial specific promoter element comprises at least one copy of the PPE-1 promoter.

29. The isolated polynucleotide of claim 26, further comprising a hypoxia response element.

30. The isolated polynucleotide of claim 29, wherein said hypoxia response element includes at least one copy of the sequence set forth in SEQ ID NO: 5.

31. A nucleic acid construct comprising the isolated polynucleotide of claim 28 and a nucleic acid sequence of interest, said nucleic acid sequence of interest being under regulatory control of said isolated polynucleotide.

32. The nucleic acid construct of claim 31, wherein said nucleic acid sequence of interest is selected from the group consisting of VEGF, p55 and PDGF-BB.

33. A mammalian cell transformed with the isolated polynucleotide of claim 28.

34. Use of a nucleic acid construct for the manufacture of a medicament for regulating angiogenesis in a tissue, said nucleic acid construct comprising:
(a) an endothelial cell specific promoter;
(b) a regulatory element including the sequence set forth in SEQ ID NO: 7.
(c) at least one copy of a hypoxia response element set forth in SEQ ID NO:5; and
(d) a nucleic acid sequence encoding an angiogenesis regulator, said nucleic acid sequence being under regulatory control of said promoter, said enhancer element and said hypoxia response element.

35. The use of claim 34, wherein said medicament is designed for administration by a method selected from the group consisting of:
(i) systemic in-vivo administration;
(ii) ex-vivo administration to cells removed from a body of a subject, and subsequent reintroduction of said cells into said body of said subject; and
(iii) local in-vivo administration.

36. The use of claim 34, wherein said endothelial cell specific promoter comprises at least one copy of the PPE-1 promoter (SEQ ID NO: 1).

37. The use of claim 34, wherein said nucleic acid sequence encoding an angiogenesis regulator is selected from the group consisting of VEGF, p55 and PDGF-BB.

38. Use of a nucleic acid construct for the manufacture of a medicament for regulating angiogenesis in a tissue, said nucleic acid construct comprising:
(a) an endothelial cell specific promoter;
(b) a regulatory element including at least one copy of the sequence set forth in SEQ ID NO:8; and
(c) a nucleic acid sequence encoding an angiogenesis regulator, said nucleic acid sequence being under regulatory control of said promoter and said regulatory element.

39. The use of claim 38, wherein said regulatory element further include at least one copy of the sequence set forth in SEQ ID NO:6.

40. The use of claim 38, wherein said regulatory element includes one copy of the sequence set forth in SEQ ID NO:8 and at least two copies of the sequence set forth in SEQ ID NO:6.

41. The use of claim 38, wherein the a nucleic acid construct further includes a hypoxia response element.

## Patentansprüche

1. Isoliertes Polynucleotid, das als ein regulatorisches Element wirkt, wobei das isolierte Polynucleotid wenigstens eine Kopie der in SEQ ID NO:8 dargelegten Sequenz umfasst, und das regulatorische Element imstande ist, die Expression einer Nucleotidsequenz zu regulieren, die mit einem Promotor, der in Endothelzellen wirkt, funktionsfähig verknüpft ist.

2. Isoliertes Polynucleotid nach Anspruch 1, wobei das regulatorische Element ferner wenigstens eine Kopie der in SEQ ID NO:6 dargelegten Sequenz umfasst.

3. Isoliertes Polynucleotid nach Anspruch 1, wobei das regulatorische Element eine Kopie der in SEQ ID NO:8 dargelegten Sequenz und wenigstens zwei Kopien der in SEQ ID NO:6 dargelegten Sequenz umfasst.

4. Isoliertes Polynucleotid nach Anspruch 1, wobei der Promotor, der in Endothelzellen wirkt, wenigstens eine Kopie des PPE-1-Promotors umfasst.

5. Isoliertes Polynucleotid nach Anspruch 1, ferner umfassend ein Hypoxia-Response-Element.

6. Isoliertes Polynucleotid nach Anspruch 5, wobei das Hypoxia-Response-Element wenigstens eine Kopie der in SEQ ID NO:5 dargelegten Sequenz umfasst.

7. Isoliertes Polynucleotid nach Anspruch 1, wobei das regulatorische Element so ist, wie in SEQ ID NO:7 dargelegt.

8. Nucleinsäurekonstrukt, umfassend das isolierte Polynucleotid nach Anspruch 1 und eine Nucleinsäuresequenz von Interesse, wobei die Nucleinsäuresequenz von Interesse unter der regulatorischen Kontrolle des isolierten Polynucleotids ist.

9. Nucleinsäurekonstrukt nach Anspruch 8, wobei die Nucleinsäuresequenz von Interesse aus der Gruppe bestehend aus VEGF, p55 und PDGF-BB ausgewählt ist,

10. Säugerzelle, transformiert mit dem isolierten Polynucleotid nach Anspruch 1.

11. Verwendung eines Konstrukts, das in Endothelzellen wirkt, zur Herstellung eines Arzneimittels für die Expression einer Nucleinsäuresequenz von Interesse in Endothelzellen, wobei das Konstrukut eine Nucleinsäuresequenz von Interesse, die unter der regulatorischen Kontrolle eines in Endothelzellen wirkenden Promotors platziert ist, und ein regulatorisches Element umfasst, das wenigstens eine Kopie der in SEQ ID NO:8 dargelegten Sequenz umfasst.

12. Verwendung nach Anspruch 11, wobei der Promotor Endtholzellspezifität aufweist.

13. Verwendung nach Anspruch 12, wobei der Promotor der PPE-1-Promotor ist, wie in SEQ ID NO:1 dargelegt.

14. Verwendung nach Anspruch 11, wobei das regulatorische Element ferner wenigstens eine Kopie der in SEQ ID NO:6 dargelegten Sequenz umfasst.

15. Verwendung nach Anspruch 14, wobei das regulatorische Element so ist, wie in SEQ ID NO:7 dargelegt.

16. Verwendung nach Anspruch 14, wobei die wenigstens eine Kopie der in SEQ ID NO:6 dargelegten Sequenz zwei Kopien umfasst.

17. Verwendung nach Anspruch 14, wobei die wenigstens zwei Kopien der in SEQ ID NO:6 dargelegten Sequenz benachbart sind.

18. Verwendung nach Anspruch 11, wobei die Nucleinsäuresequenz aus der Gruppe bestehend aus VEGF, p55 und PDGF-BB ausgewählt ist.

19. Verwendung nach Anspruch 11, wobei das Arzneimittel zur Verabreichung durch ein Verfahren ausgelegt ist, das ausgewählt ist aus der Gruppe bestehend aus:
(i) systemische in-vivo-Verabreichung;
(ii) ex-vivo-Verabreichung an Zellen, die einem Körper eines Probanden entnommen sind, und anschließende Wiedereinführung der Zellen in den Körper des Probanden; und
(iii) lokale in-vivo-Verabreichung.

20. Verwendung eines Nucleinsäurekonstrukts zur Herstellung eines Arzneimittels zum Regulieren der Angiogenese in einem Gewebe, wobei das Nucleinsäurekonstrukt umfasst:
(a) einen endothelzellspezifischen Promotor;
(b) wenigstens eine Kopie eines in SEQ ID NO:5 dargelegten Hypoxia-Response-Elements;
(c) ein regulatorisches Element, das wenigstens eine Kopie der in SEQ ID NO:8 dargelegten Sequenz umfasst; und
(d) eine Nucleinsäuresequenz, die einen Angiogenese-Regulator codiert, wobei die Nucleinsäuresequenz unter der regulatorischen Kontrolle des Promotors und des Hypoxia-Response-Elements ist.

21. Verwendung nach Anspruch 20, wobei das Arzneimittel zur Verabreichung durch ein Verfahren ausgelegt ist, das ausgewählt ist aus der Gruppe bestehend aus:
(i) systemische in-vivo- Verabreichung;
(ii) ex-vivo-Verabreichung an Zellen, die einem Körper eines Probanden entnommen sind, wobei die Zellen anschließend wieder in den Körper des Probanden eingeführt werden; und
(iii) lokale in-vivo-Verabreichung.

22. Verwendung nach 20, wobei das Nucleinsäurekonstrukt ferner umfasst:
(e) wenigstens eine Kopie einer in SEQ ID NO:6 dargelegten Sequenz.

23. Verwendung nach Anspruch 22, wobei die wenigstens eine Kopie einer in SEQ ID NO:6 dargelegtenn Sequenz zwei benachbarte Kopien umfasst.

24. Verwendung nach Anspruch 20, wobei der endothelzellspezifische Promotor wenigstens eine Kopie des PPE-1-Promotors umfasst.

25. Verwendung nach Anspruch 20, wobei die Nucleinsäuresequenz, die einen Angiogenese-Regulator codiert, aus der Gruppe bestehend aus VEGF, p55 und PDGF-BB ausgewählt ist.

26. Isoliertes Polynucleotid, das als ein Promotor in eukaryotischen Zellen wirkt, wobei das isolierte Polynulceotid ein regulatorisches Element umfasst, das die in SEQ ID NO:7 dargelegte Sequenz umfasst.

27. Isoliertes Polynucleotid nach Anspruch 26, ferner umfassend ein endothelzellspezifisches Promotor-Element.

28. Isoliertes Polynucleotid nach Anspruch 27, wobei das endotheizellspezifische Promotor-Element wenigstens eine Kopie des PPE-1-Promotors umfasst.

29. Isoliertes Polynucleotid nach Anspruch 26, ferner umfassend ein Hypoxia-Response-Element.

30. Isoliertes Polynucleotid nach Anspruch 29, wobei das Hypoxia-Response-Element wenigstens eine Kopie der in SEQ ID NO:5 dargelegten Sequenz umfasst.

31. Nucleinsäurekonstrukt, umfassend das isolierte Polynucleotid nach Anspruch 28 und eine Nucleinsäuresequenz von Interesse, wobei die Nucleinsäuresequenz von Interesse unter der regulatorischen Kontrolle des isolierten Polynucleotids ist.

32. Nucleinsäurekonstrukt nach Anspruch 31, wobei die Nucleinsäuresequenz von Interesse aus der Gruppe bestehend aus VEGF, p55 und PDGF-BB ausgewählt ist.

33. Säugerzelle, transformiert mit dem isolierten Polynucleotid nach Anspruch 28.

34. Verwendung eines Nucleinsäurekonstrukts zur Herstellung eines Arzneimittels zum Regulieren der Angiogenese in einem Gewebe, wobei das Nucleinsäurekonstrukt umfasst:
(a) einen endothelzellspezifischen Promotor;
(b) ein regulatorisches Element, das die in SEQ ID NO:7 dargelegte Sequenz umfasst;
(c) wenigstens eine Kopie eines in SEQ ID NO:5 dargelegten Hypoxia-Response-Elements; und
(d) eine Nucleinsäuresequenz, die einen Angiogenese-Regulator codiert, wobei die Nucleinsäuresequenz unter der regulatorischen Kontrolle des Promotors, des Enhancer-Elements und des Hypoxia-Response-Elements ist.

35. Verwendung nach Anspruch 34, wobei das Arzneimittel zur Verabreichung durch ein Verfahren ausgelegt ist, das ausgewählt ist aus der Gruppe bestehend aus:
(i) systemische in-vivo-Verabreichung;
(ii) ex-vivo-Verabreichung an Zellen, die einem Körper eines Probanden entnommen sind, und anschließende Wiedereinführung der Zellen in den Körper des Probanden; und
(iii) lokale in-vivo-Verabreichung.

36. Verwendung nach Anspruch 34, wobei der endothelzellspezifische Promotor wenigstens eine Kopie des PPE-I-Promotors (SEQ ID NO:1) umfasst.

37. Verwendung nach 34, wobei die Nucleinsäuresequenz, die einen Angiogenese-Regulator codiert, aus der Gruppe bestehend aus VEGF, p55 und PDGF-BB ausgewählt ist.

38. Verwendung eines Nucleinsäurekonstrukts zur Herstellung eines Arzneimittels zum Regulieren der Angiogenese in einem Gewebe, wobei das Nucleinsäurekonstrukt umfasst:
(a) einen endothelzellspezifischen Promotor;
(b) ein regulatorisches Element, das wenigstens eine Kopie der in SEQ ID NO:8 dargelegten Sequenz umfasst;
(c) eine Nucleinsäuresequenz, die einen Angiogenese-Regulator codiert, wobei die Nucleinsäuresequenz unter der regulatorischen Kontrolle des Promotors und des regulatorischen Elements ist.

39. Verwendung nach Anspruch 38, wobei das regulatorische Element ferner wenigstens eine Kopie der in SEQ ID NO:6 dargelegten Sequenz umfasst.

40. Verwendung nach Anspruch 38, wobei das regulatorische Element eine Kopie der in SEQ ID NO:8 dargelegten Sequenz und wenigstens zwei Kopien der in SEQ ID NO:6 dargelegten Sequenz umfasst.

41. Verwendung nach Anspruch 38, wobei das Nucleinsäurekonstrukt ferner ein Hypoxia-Response-Element umfasst.

## Revendications

1. Polynucléotide isolé fonctionnel en tant qu'élément régulateur, le polynucléotide isolé comprenant au moins une copie de la séquence décrite dans SEQ ID NO: 8, ledit élément régulateur étant capable de réguler l'expression d'une séquence de nucléotides en liaison fonctionnelle avec un promoteur fonctionnel dans des cellules endothéliales.

2. Polynucléotide isolé selon la revendication 1, où ledit élément régulateur comprend en outre au moins une copie de la séquence décrite dans SEQ ID NO: 6.

3. Polynucléotide isolé selon la revendication 1, où ledit élément régulateur comprend une copie de la séquence décrite dans SEQ ID NO: 8 et au moins deux copies de la séquence décrite dans SEQ ID NO: 6.

4. Polynucléotide isolé selon la revendication 1, où ledit promoteur fonctionnel dans des cellules endothéliales comprend au moins une copie du promoteur PPE-1.

5. Polynucléotide isolé selon la revendication 1, comprenant en outre un élément de réponse à l'hypoxie.

6. Polynucléotide isolé selon la revendication 5, où ledit élément de réponse à l'hypoxie comprend au moins une copie de la séquence décrite dans SEQ ID NO: 5.

7. Polynucléotide isolé selon la revendication 1, où ledit élément régulateur est tel que décrit dans SEQ ID NO: 7.

8. Produit de recombinaison d'acide nucléique comprenant le polynucléotide isolé de la revendication 1 et une séquence d'acide nucléique d'intérêt, ladite séquence d'acide nucléique d'intérêt étant sous le contrôle régulateur dudit polynucléotide isolé.

9. Produit de recombinaison d'acide nucléique selon la revendication 8, où ladite séquence d'acide nucléique d'intérêt est sélectionnée dans le groupe consistant en VEGF, p55 et PDGF-BB.

10. Cellule mammalienne transformée avec le polynucléotide isolé de la revendication 1.

11. Utilisation d'un produit de recombinaison fonctionnel dans des cellules endothéliales pour la fabrication d'un médicament pour l'expression d'une séquence d'acide nucléique d'intérêt dans des cellules endothéliales, ledit produit de recombinaison comprenant une séquence d'acide nucléique d'intérêt positionnée sous le contrôle régulateur d'un promoteur fonctionnel dans des cellules endothéliales, et un élément régulateur comprenant au moins une copie de la séquence décrite dans SEQ ID NO: 8.

12. Utilisation selon la revendication 11, où ledit promoteur exhibe une spécificité pour des cellules endothéliales.

13. Utilisation selon la revendication 12, où ledit promoteur est le promoteur PPE-1 tel que décrit dans SEQ ID NO: 1.

14. Utilisation selon la revendication 11, où ledit élément régulateur comprend en outre au moins une copie de la séquence décrite dans SEQ ID NO: 6.

15. Utilisation selon la revendication 14, où ledit élément régulateur est tel que décrit dans SEQ ID NO: 7.

16. Utilisation selon la revendication 14, où ladite au moins une copie de la séquence décrite dans SEQ ID NO: 6 comprend deux copies.

17. Utilisation selon la revendication 14, où lesdites au moins deux copies de la séquence décrite dans SEQ ID NO: 6 sont contiguës.

18. Utilisation selon la revendication 11, où ladite séquence d'acide nucléique d'intérêt est sélectionnée dans le groupe consistant en VEGF, p55 et PDGF-BB,

19. Utilisation selon la revendication 11, où ledit médicament est conçu pour une administration par un procédé sélectionné dans le groupe consistant en :
(i) une administration *in vivo* systémique ;
(ii) une administration *ex vivo* à des cellules prélevées du corps d'un sujet puis une réintroduction ultérieure desdites cellules dans ledit corps dudit sujet ; et
(iii) une administration *in vivo* locale.

20. Utilisation d'un produit de recombinaison d'acide nucléique pour la fabrication d'un médicament destiné à réguler l'angiogenèse dans un tissu, le produit de recombinaison d'acide nucléique comprenant :
(a) un promoteur spécifique aux cellules endothéliales ;
(b) au moins une copie d'un élément de réponse à l'hypoxie tel que décrit dans SEQ ID NO: 5 ;
(c) un élément régulateur comprenant au moins une copie de la séquence décrite dans SEQ ID NO: 8, et
(d) une séquence d'acide nucléique codant pour un régulateur de l'angiogenèse, ladite séquence d'acide nucléique étant sous le contrôle régulateur dudit promoteur et dudit élément de réponse à l'hypoxie.

21. Utilisation selon la revendication 20, où ledit médicament est conçu pour une administration par un procédé sélectionné dans le groupe consistant en :
(i) une administration *in vivo* systémique ;
(ii) une administration *ex vivo* à des cellules prélevées du corps d'un sujet, lesdites cellules étant ultérieurement réintroduites dans ledit corps dudit sujet ; et
(iii) une administration *in vivo* locale.

22. Utilisation selon la revendication 20, où ledit produit de recombinaison d'acide nucléique comprend en outre :
(e) au moins une copie d'une séquence décrite dans SEQ ID NO: 6.

23. Utilisation selon la revendication 22, où ladite au moins une copie d'une séquence décrite dans SEQ ID NO: 6 comprend deux copies contigues.

24. Utilisation selon la revendication 20, où ledit promoteur spécifique aux cellules endothéliales comprend au moins une copie du promoteur PPE-1,

25. Utilisation selon la revendication 20, où ladite séquence d'acide nucléique codant pour un régulateur de l'angiogenèse est sélectionnée dans le groupe consistant en le VEGF, p55 et PDCaF-BB.

26. Polynucléotide isolé fonctionnel en tant que promoteur dans des cellules eucaryotes, le polynucléotide isolé comprenant un élément régulateur comprenant la séquence décrite dans SEQ ID NO: 7.

27. Polynucléotide isolé selon la revendication 26, comprenant en outre un élément promoteur spécifique aux cellules endothéliales.

28. Polynucléotide isolé selon la revendication 27, où ledit élément promoteur spécifique aux cellules endothéliales comprend au moins une copie du promoteur PPE-1.

29. Polynucléotide isolé selon la revendication 26, comprenant en outre un élément de réponse à l'hypoxie.

30. Polynucléotide isolé selon la revendication 29, où ledit élément de réponse à l'hypoxie comprend au moins une copie de la séquence décrite dans SEQ ID NO: 5.

31. Produit de recombinaison d'acide nucléique comprenant le polynucléotide isolé de la revendication 28 et une séquence d'acide nucléique d'intérêt, ladite séquence d'acide nucléique d'intérêt étant sous le contrôle régulateur dudit polynucléotide isolé.

32. Produit de recombinaison d'acide nucléique selon la revendication 31, où ladite séquence d'acide nucléique d'intérêt est sélectionnée dans le groupe consistant en le VEGF, p55 et PDGF-BB.

33. Cellule mammalienne transformée avec le polynucléotide isolé de la revendication 28.

34. Utilisation d'un produit de recombinaison d'acide nucléique pour la fabrication d'un médicament destiné à réguler l'angiogenèse dans un tissu, ledit produit de recombinaison d'acide nucléique comprenant :
(a) un promoteur spécifique aux cellules endothéliales ;
(b) un élément régulateur comprenant la séquence décrite dans SEQ ID NO: 7.
(c) au moins une copie d'un élément de réponse à l'hypoxie tel que décrit dans SEQ ID NO; 5 ; et
(d) une séquence d'acide nucléique codant pour un régulateur de l'angiogenèse, ladite séquence d'acide nucléique étant sous le contrôle régulateur dudit promoteur, dudit élément amplificateur et dudit élément de réponse à l'hypoxie.

35. Utilisation selon la revendication 34, où ledit médicament est conçu pour une administration par un procédé sélectionné dans le groupe consistant en :
(i) une administration *in vivo* systémique ;
(ii) une administration *ex vivo* à des cellules prélevées du corps d'un sujet, puis une réintroduction ultérieure desdites cellules dans ledit corps dudit sujet ; et
(iii) une administration *in vivo* locale.

36. Utilisation selon la revendication 34, où ledit promoteur spécifique aux cellules endothéliales comprend au moins une copie du promoteur PPE-1 (SEQ ID NO: 1).

37. Utilisation selon la revendication 34, où ladite séquence d'acide nucléique codant pour un régulateur de l'angiogenèse est sélectionnée dans le groupe consistant en le VEGF, p55 et PDGF-BB,

38. Utilisation d'un produit de recombinaison d'acide nucléique pour la fabrication d'un médicament destiné à réguler l'angiogenèse dans un tissu, ledit produit de recombinaison d'acide nucléique comprenant :
(a) un promoteur spécifique aux cellules endothéliales ;
(b) un élément régulateur comprenant au moins une copie de la séquence décrite dans SEQ ID NO: 8 ; et
(c) une séquence d'acide nucléique codant pour un régulateur de l'angiogenèse, ladite séquence d'acide nucléique étant sous le contrôle régulateur dudit promoteur et dudit élément régulateur.

39. Utilisation selon la revendication 38, où ledit élément régulateur comprend en outre au moins une copie de la séquence décrite dans SEQ ID NO: 6.

40. Utilisation selon la revendication 38, où ledit élément régulateur comprend une copie de la séquence décrite dans SEQ ID NO: 8 et au moins deux copies de la séquence décrite dans SEQ ID NO: 6.

41. Utilisation selon la revendication 38, où le produit de recombinaison d'acide nucléique comprend en outre un élément de réponse à l'hypoxie.
